# EUROPEAN PATENT APPLICATION

(11) **EP 0 403 739 A2**
(43) Date of publication of application: **27.12.1990**
(21) Application number: 90105818.0
(22) Date of filing: 27.03.1990
(51) Int. Cl.: C07C 69/76, C07C 233/78, C07C 309/74, C07C 311/18, C07C 43/164, C07C 211/61, C07C 15/28, G11B 7/24, G02F 3/00, G02F 1/01

(54) **Photosensitive compounds and their preparation and use**

(30) Priority: 27.03.1989 JP 71718/89
(71) Applicant: HITACHI, LTD., Chiyoda-ku, Tokyo 100 (JP)
(72) Inventor: Sasaki, Hiroshi, Naka-gun, Ibaraki-ken (JP); Kobayashi, Setsuo, Hitachi-shi, Ibaraki-ken (JP); Iwasaki, Kishiro, Hitachiohta-shi, Ibaraki-ken (JP); Nakamura, Mariko, Hitachi-shi, Ibaraki-ken (JP); Ito, Yutaka, Takahagi-shi, Ibaraki-ken (JP); Mukoo, Akio, Mito-shi, Ibaraki-ken (JP); Osa, Tetsuo, Sendai-shi, Miyagi-ken (JP); Ueno, Akihiko, Sendai-shi, Miyagi-ken (JP)
(74) Representative: Patentanwälte Beetz - Timpe - Siegfried Schmitt-Fumian - Mayr

(57) **Abstract**

The present invention relates to photosensitive compounds which are capable of producing trans-dimerization between two aromatic ring residues in the molecule so as to allow light of 300 nm or more to pass therethrough when irradiated with ultraviolet light of 340 nm to 380 nm, and which are also capable of cutting off the passage of light of 300 nm or more when the dimerization is dissociated by irradiation with ultraviolet light of 240 to 280 nm. The invention also relates to methods for preparing these compounds and to photosensitive elements, optical circuits, optical recording media, and the like, which contain the photosensitive compounds as components.

## Description

The present invention relates to novel photosensitive compounds, and particularly to photosensitive compounds which are capable of producing trans-dimerization between two aromatic ring residues in their molecule when irradiated with ultraviolet light of 340 to 380 nm, the dimerization being dissociated by irradiation with ultraviolet light of 240 to 280 nm. The present invention also relates to methods of their preparation, and their use in or for photosensitive members, an optical recording media, optical memory elements, optical circuits, optical switches, optical logical elements, optical filters and very high speed optical shutters.

Various compounds have been reported as being reversible optical recording organic materials. Of these compounds, compounds such as azobenzene, fulgide and the like, which generally exhibit excellent repeatability, require much time for isomerization. Spiropyran derivatives, which are relatively rapidly isomerized, exhibit poor repeatability. Since these photochromic compounds produce reversible photoisomerization reaction when irradiated with ultraviolet and visible light, when a disk comprising one of such compounds and having data written by ultraviolet light is accidentally allowed to stand under visible light, there is the danger that the written record could be erased.

Photochromic compounds such as naphthalene and anthracene having condensed polycyclic hydrocarbon groups are capable of producing and dissociating dimerization when irradiated with two kinds of ultraviolet light having different wavelengths. However, conventional compounds exhibiting this phenomenon undergo cis-type dimerization reaction. These compounds also have problems with respect to their preservation stability because dimerization is dissociated by heat. This kind of compounds constitutes a cyclophane having two condensed polycyclic hydrocarbons groups in their skeleton, are disclosed in JP-A-57-147630 and JP-A-60-54374.

It has been reported that a trans dimer of anthracene is not easily dissociated to its original state by heat (Journal of Chemical Society, Chemical Communication, 1042, 1988).

Substantially none of the above-described optical recording organic materials can be synthesized in large quantities in a short time at low cost.

The aforementioned conventional compounds involve problems with respect to characteristics such as repeatability, recording speed, recording stability, and the problem that they cannot be synthesized in large quantities in a short time at low cost.

Accordingly, it is an object of the present invention to provide photosensitive compounds, photochromic compounds and reversible optical recording materials and their preparation, which exhibit improved repeatability when irradiated with ultraviolet rays, improved thermal recording stability and increased recording speed and which can be easily synthesized, and to provide materials useful for optical computer circuit elements such as optical recording media, optical memory elements, optical switches and optical logical elements, very high speed shutters and optical filters, which use the above compounds.

The above object is achieved according to the claims. The dependent claims relate to preferred embodiments.

The present invention provides photosensitive compounds and photochromic compounds which have two condensed polycyclic hydrocarbon groups at photoresponse positions and which produce trans-type dimerization reaction and are thermally stable, and also provides optical computer circuit elements such as a reversible optical recording media, optical memory elements, optical switches and optical logical elements, very high speed optical shutters and optical filters, which use the above compounds.

The photosensitive compounds of the present invention have the general formula I, 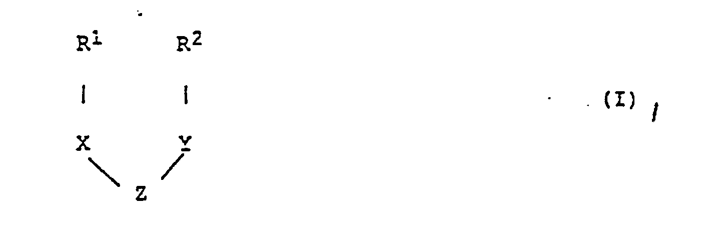 wherein denote:
- R' and R² independently an optionally substituted residue of naphthalene, anthracene, phenanthrene, triphenylene, chrysene, naphthacene, benzoanthracene, picene, pentaphene, pentacene or dibenzophenan- threne, where R¹ and R² may be the same if neither R' nor R² is a naphthalene residue;
- X and Y independently an ester bond, a thioester bond, an amide bond, a thioamide bond, an ether bond, a thioether bond, a sulfonyloxy bond, a sulfonylamino bond, an amine bond, or a methylene or ethylene bond, and
- Z 4 CH₂ )̵ₘ(wherein m denotes an integer of 6 to 16), -CH₂(̵CH₂OCH₂)̵_{m̅}, CH₂- (wherein m' denotes an integer of 3 to 10), -CH₂(̵CH₂)̵ₙO(̵CH_{2 n}CH₂- (wherein n and n each denote an integer of 2 to 20), or a phenylene group, a xylylene group or a cycloalkane residue having 6 or more carbon atoms, Z optionally being substituted.

The compounds of the formula III are capable of producing dimerization between the aromatic ring residues in their molecule when irradiated with ultraviolet light of 340 to 380 nm, the dimerization being dissociated by irradiation with ultraviolet light of 240 to 280 nm, i.e., the compounds of formula |I| have photochromic properties.

The compounds of formula |I| of the present invention are characterized by trans-type dimerization produced by irradiation with light of 340 to 380 nm. Thus, the redissociation of the dimerization is not easily achieved only by applying thermal energy, and the thermal stability of retention of the dimerization is extremely excellent, as compared with conventional photochromic compounds.

Although the reversible optical reaction in the present invention is mainly characterized by selective progress using ultraviolet light, active radiation such as heat radiation, X-rays, electron rays, m-wave rays, infrared rays or the like may be used in combination with ultraviolet rays, as occasion demands.

In the present invention, examples of the compounds of formula |I| include the following compound groups (A) to (I);
Compound group (A); R¹= R²: anthracene derivative X = Y: ester bond 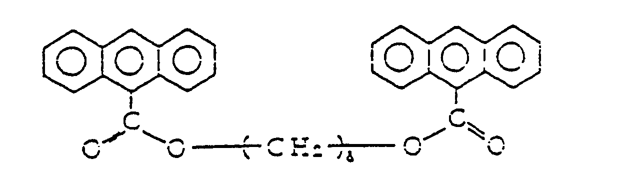 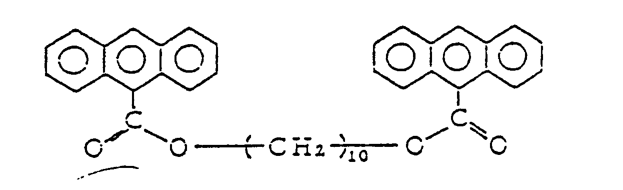 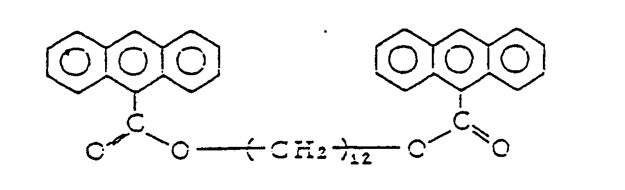 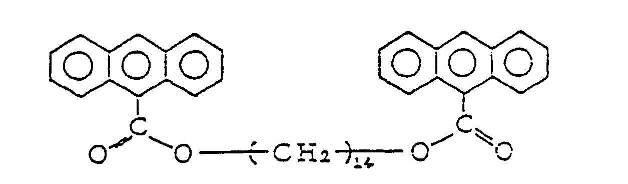 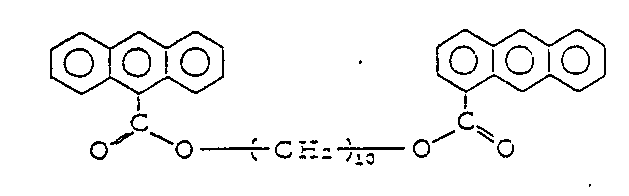 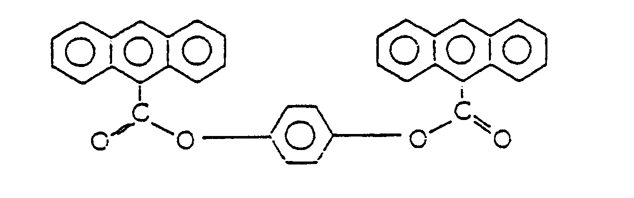 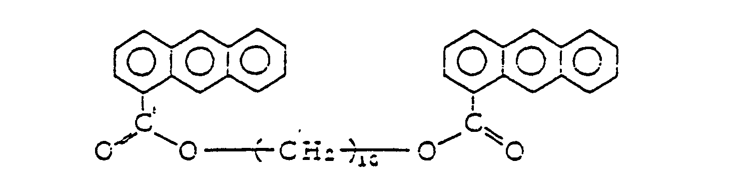 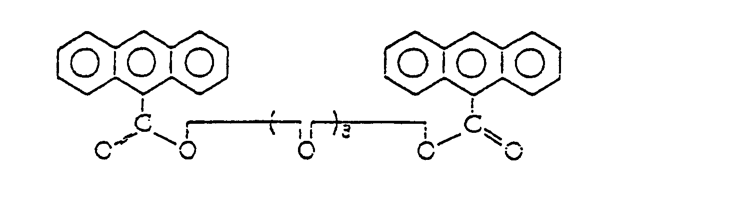 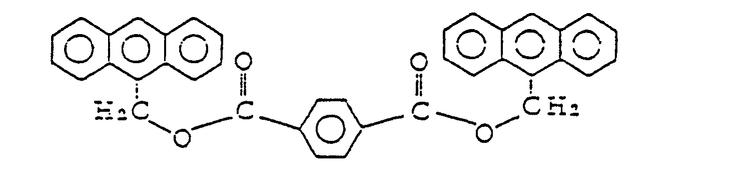 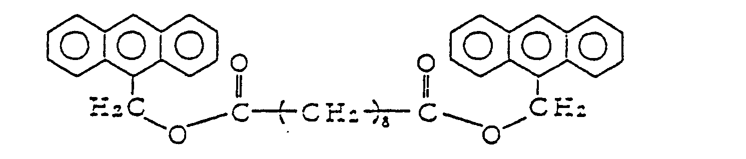 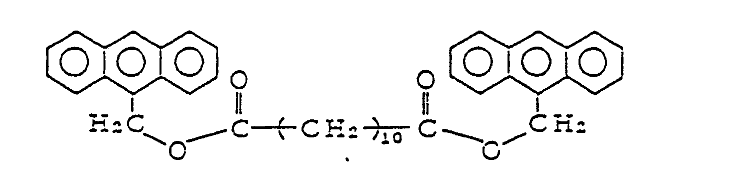 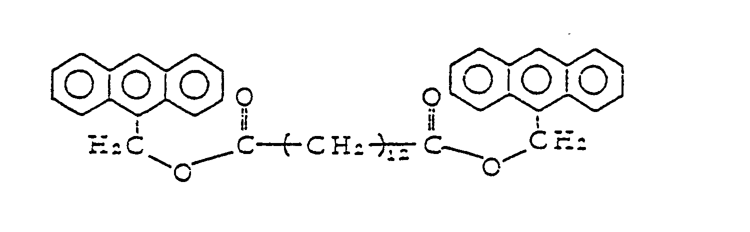 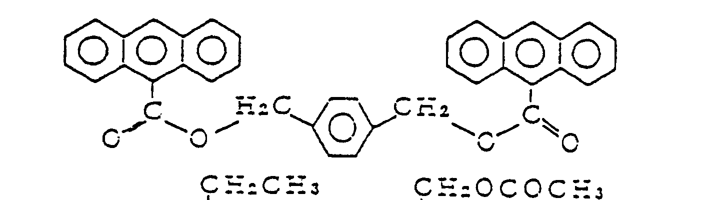 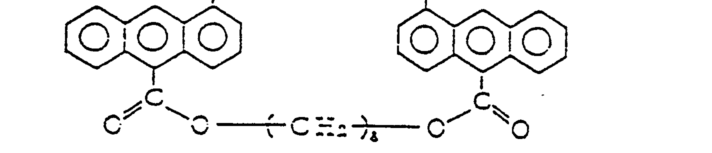 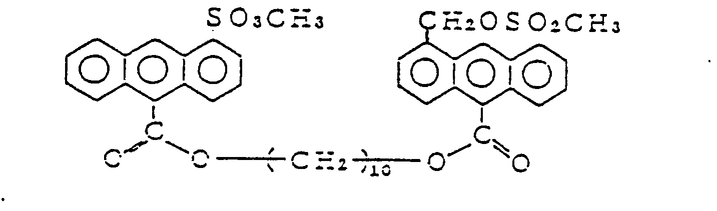 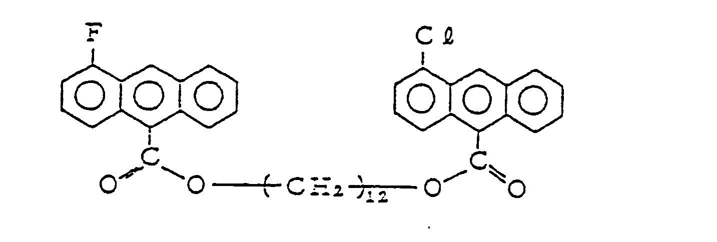 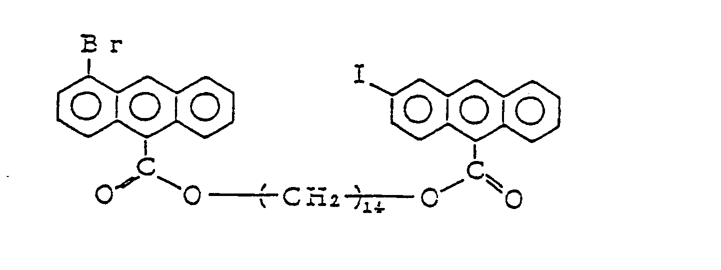 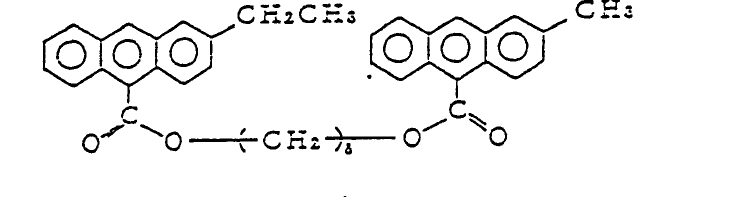 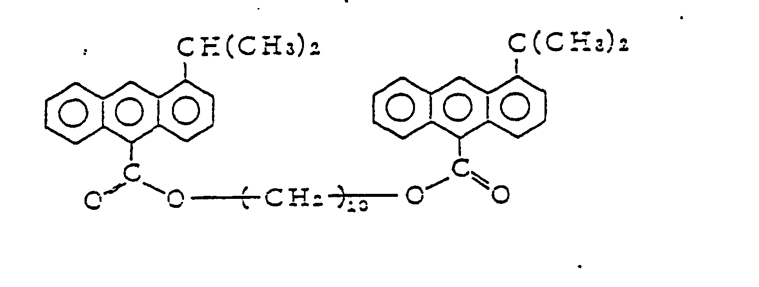 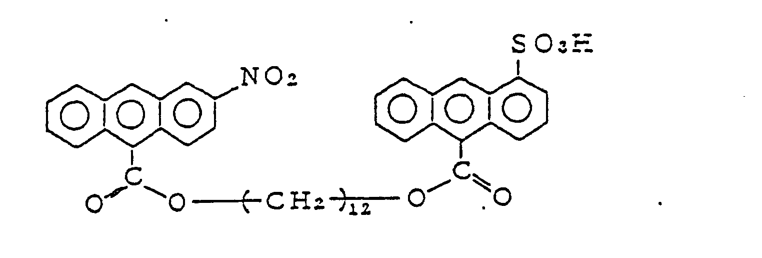 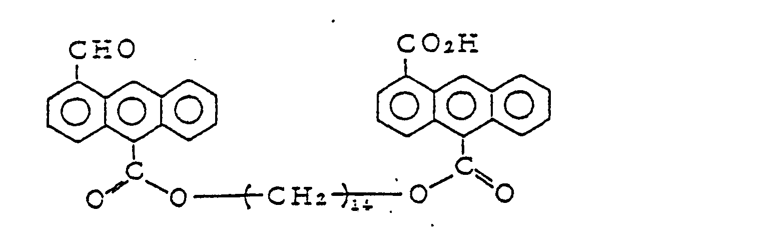
Compound group (B) ; R¹: naphthalene, R²: anthracene, X = Y: ester bond 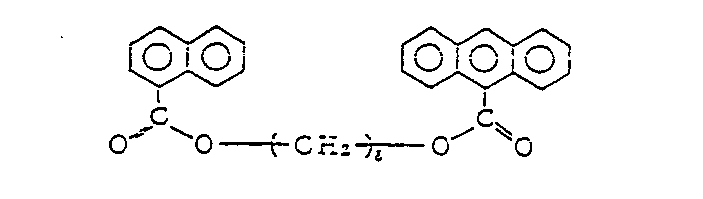 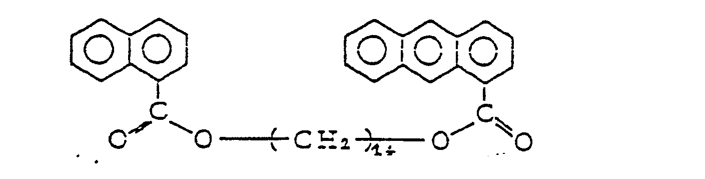 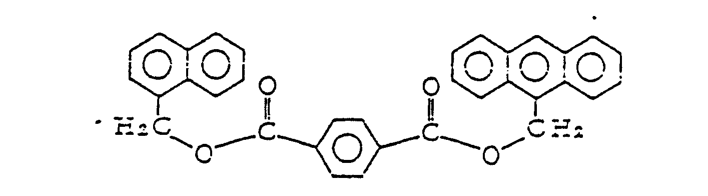 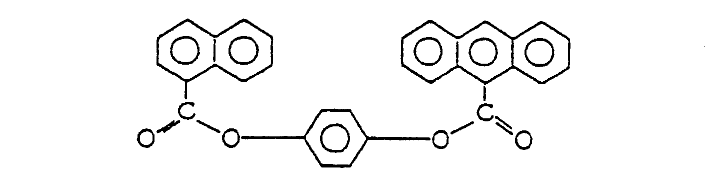 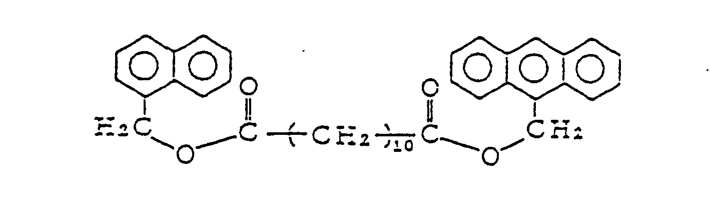 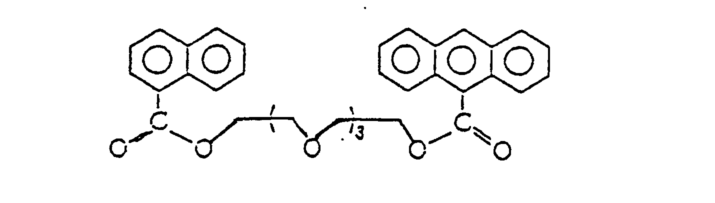 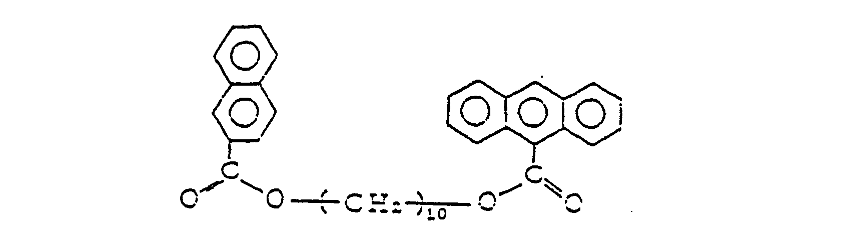
Compound group (C) ; R^{l}: naphthalene, R²: anthracene, X = Y: ester bond 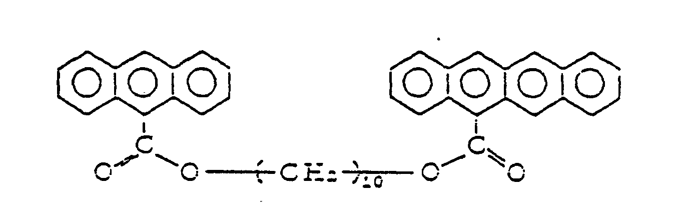 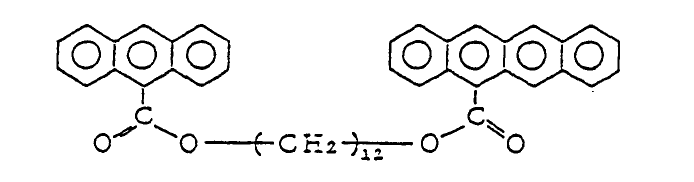 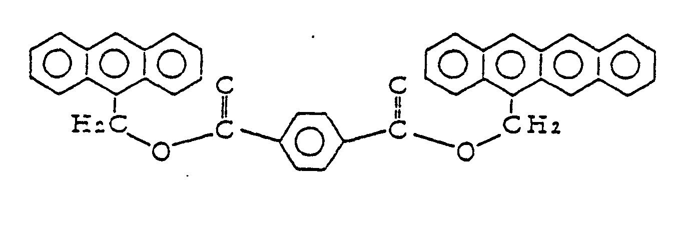 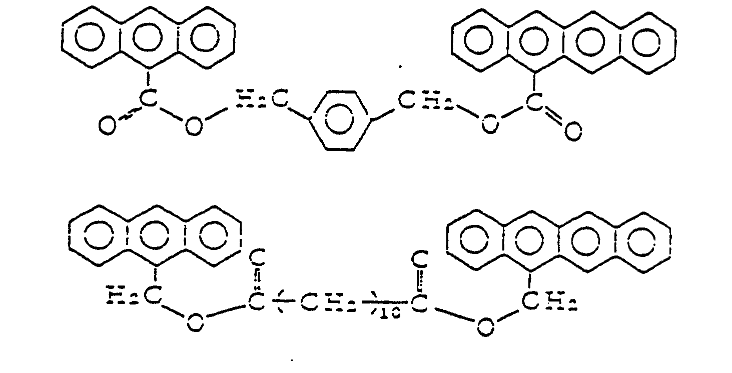
Compound group (D); R¹ = R²: anthracene, X = Y: amide bond 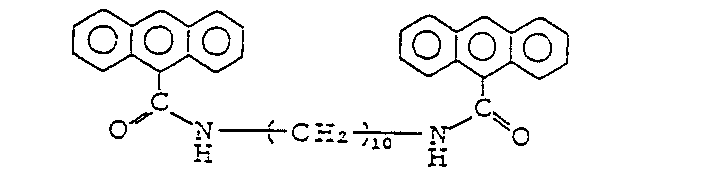 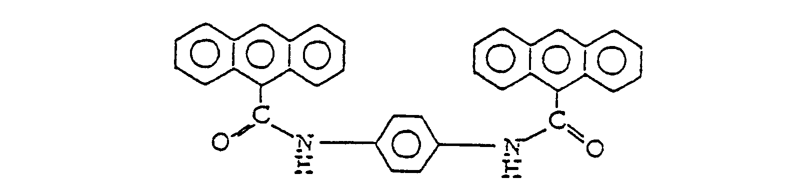
Compound group (E); R^{l}: naphthalene, R² : anthracene, 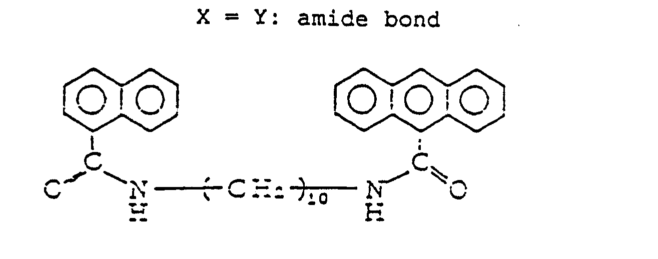 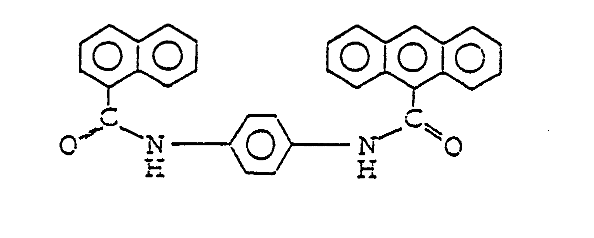
Compound group (F); R¹ = R²: anthracene, X = Y: thioester bond 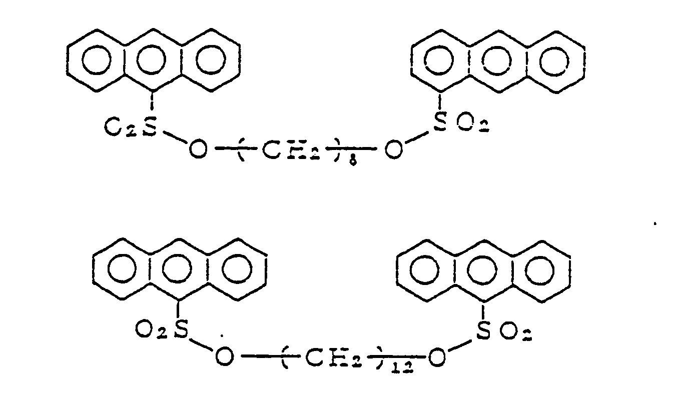
Compound group (G); R¹ = R²: anthracene, X = Y: ether bond 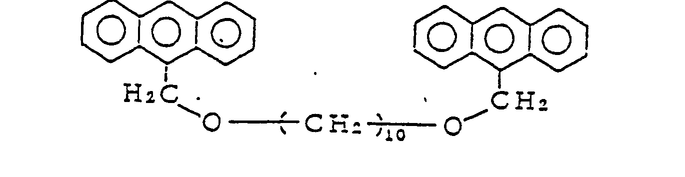 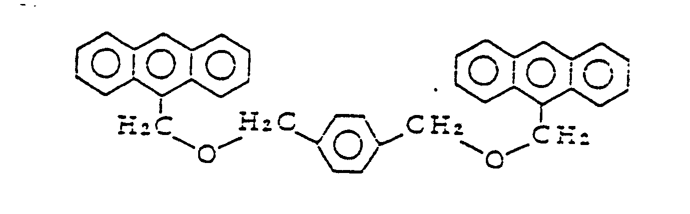
Compound group (H); R¹ = R²: anthracene, X = Y: amine bond 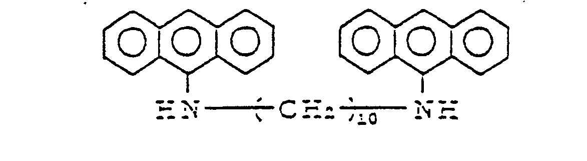 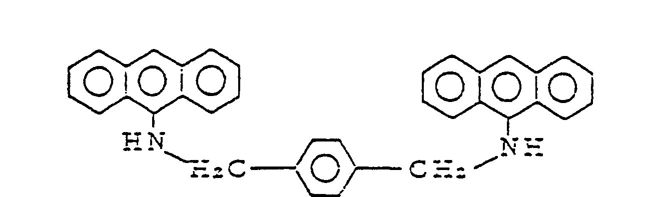
Compound group (I); R¹ = R²: anthracene, X = Y: methylene bond

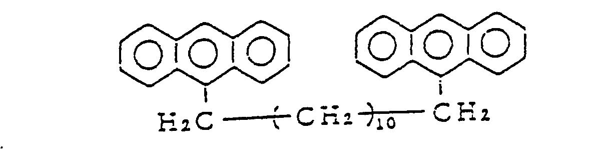

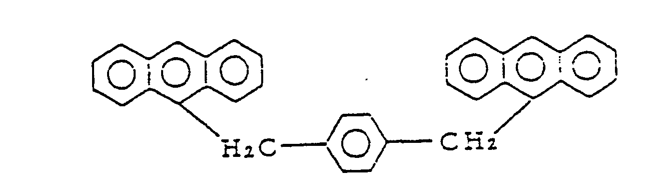

The above-described compounds include compounds expressed by formulae (II-a), (II-b), (II-c) and (II-d) in the present invention.

The above-described compounds also include compounds expressed by formulae (III-a), (III-b), (III-c), (III-d) and (III-e) in the present invention. 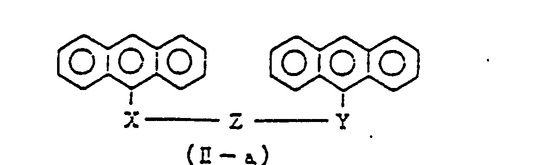 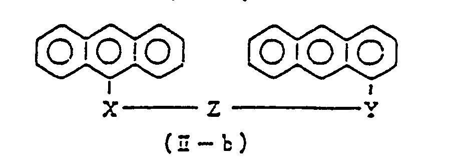 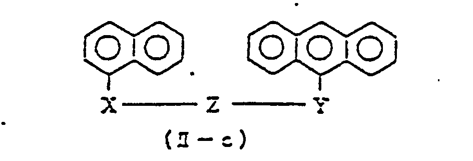 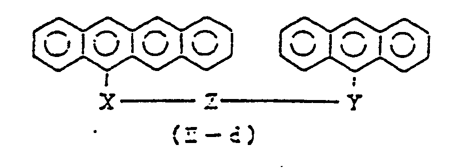 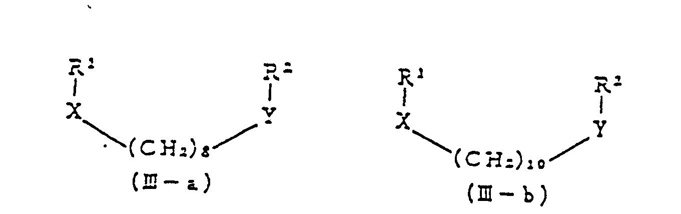 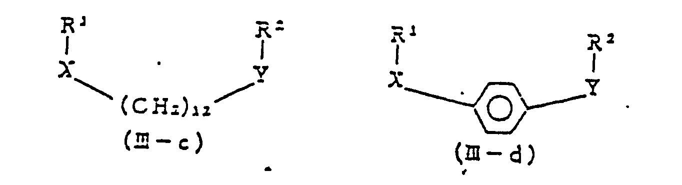 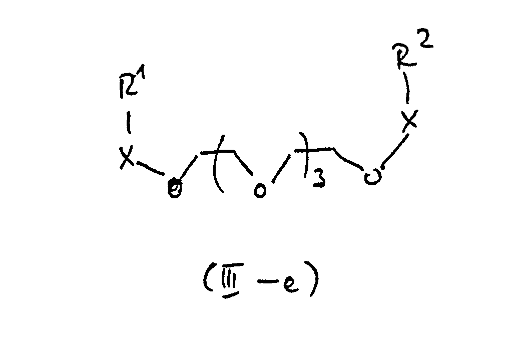 Wherein R¹, R², X, Y and Z are as defined above.

The invention also specifically covers photosensitive compounds of the following formula IVa to IVe: 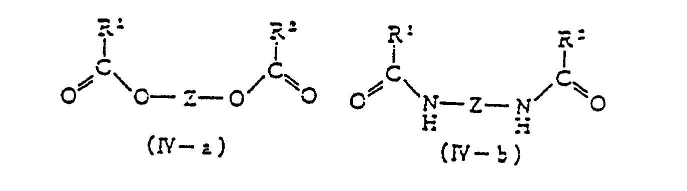 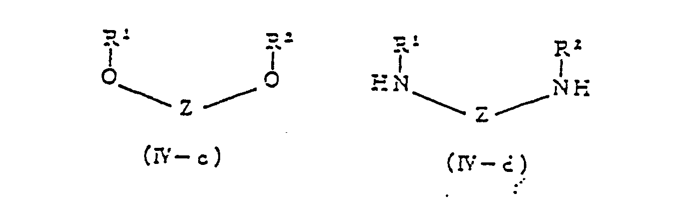 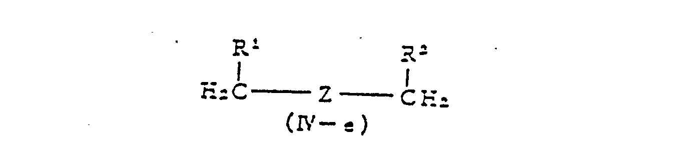 Wherein R¹, R² and Z are as defined above.

The method of the present invention for preparing the photosensitive compounds of formula I is characterized by
(A) reading a compound of the general formula IVa or IVb R¹ - R³ (IVa) or R^{2 -} R³ (IVb), wherein
   R¹ and R² are as defined above, and
   R³ is -CO-OH, -CO-SH, -CS-OH, -S0₂-OH, -S0₂-Hal (Hal denoting halogen), -CO-Hal, or -CS-Hal,
   or a reactive derivative of these groups, with a compound of the general formula V
   R^{4 -} Z - R⁴ (V), wherein
   Z is as defined above and
   R⁴ is -OH, -NH₂, -Hal, or -SH,
   or a reactive derivative of these groups, or
(B) reading a compound of the general formula Via or Vlb R' - R⁵ (Via) or R^{2 -} R⁵ (Vlb) ,
   wherein R¹ and R² are as defined above and
   R⁵ is -CH₂-OH, -CH₂-Hal, -CH₂-SH, or -CH₂-NH₂,
   or a reactive derivative of these groups; with a compound of the general formula (VII) R⁶- Z - R⁶ (VII) , wherein Z is as defined above, and
   R⁶ is -COOH, -CO-SH, -CS-OH, -S0₂-OH, -S0₂-Hal, -CO-Hal, -CS-Hal, or -NH₂,
   or a reactive derivative of these groups, or
(C) reading a compound of the general formula Vllla or Vlllb 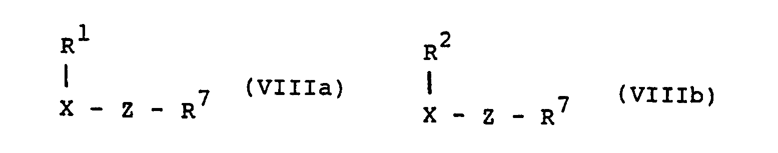 wherein
   R¹, R² and Z are as defined above, and
   R⁷ is -OH, -Hal, or -NH₂, -SH,
   or a reactive derivative thereof, with a compound of the general formula IVa or IVb, R¹ - R³ (IVa) or R^{2 -} R³ (IVb) as defined above, or
(D) reading a compound of the general formula IXa or IXb, 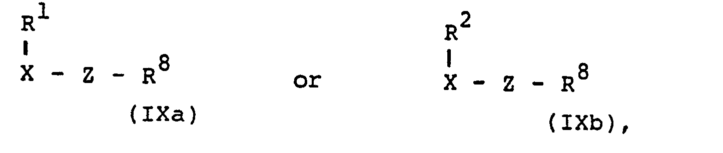 wherein
   R¹, R² and Z are as defined above, and
   R⁸ is -CO-OH, -CO-SH, -CS-OH, -S0₂-OH, -S0₂-Hal, -CO-Hal, or -CS-Hal,
   or a reactive derivative of these groups, with a compound of the general formula Xa or Xb,
   _{R' - R4 (Xa)} or _{R2} - _{R}⁴ (Xb), wherein
   R¹, R² and R⁴ are as defined as above.

More specifically, for the case of the anthracene and phenylene groups, the compounds of formula III can be obtained, for example, by the following methods (1) to (5):
(1) Condensation of an anthracene monocarboxylic acid and a long-chain alkyl diol or a dioxybenzene (benzenediol);
(2) Condensation of an anthracene monocarboxylic acid and a long-chain alkyl diamine or a diaminobenzene;
(3) Condensation of anthracene methanol (hydroxymethylanthracene) and a long-chain or benzene dicarboxylic acid;
(4) Condensation of anthracene methanol and a long-chain dihalide or a dihalogenobenzene, or
(5) Condensation of anthracene methanol and a long-chain diamine or a diaminobenzene.

Although typical synthesis conditions are described in detail in the examples below, the methods of the present invention are not particularly limited thereto.

In the present invention, when one of the compounds expressed by the formula III is used as a photosensitive element or member such as a reversible recording medium or the like, an appropriate polymer is generally used as a binder in the form of a dispersion, but the compounds can be singly used.

Examples of binders are acrylic resins, cellulose resins, polyamide resins, polyester resins, polyvinyl formal, polyvinyl butyral, polyvinyl alcohol, polystyrene, polyvinyl chloride, vinyl chloride, polyvinyl acetate, gelatin, phenoxy resins, urethane resins, siloxane resins, epoxy resins, phenolic resins, melamine resins, urea resins, addition reaction-type imide resins and the like.

Any materials containing the compounds expressed by the formula III may be used as recording materials in the present invention. A typical form of the recording materials is a form in which one of the compounds is provided on, for example, paper, a film or a metallic foil, which serves as a base material. Another form, that can be used, is a form in which a solution of one of the compounds is sealed between transparent materials such as glass, quartz or resin plates.

The recording materials of the present invention are generally produced in the form of a film by a conventional method of producing recording materials such as an application method using a coater, impregnation or lamination dipping.

A protective surface layer can be also provided on the recording material, as occasion demands. The recording materials of the present invention can contain various additives such as stabilizers, photosensitizers, light absorbers, coating materials, fillers and the like in accordance with the purpose of use.

The present invention further provides photosensitive members or elements composed of a polymeric compound which transmits light having a wavelength of 300 nm or more and which contains a photosensitive compounds expressed by the formula |I|. The method of producing these members comprises the steps of forming a solution by dissolving (in a solvent) a polymeric compound, which transmits light having a wavelength of 300 nm or more, and a photosensitive compound, which produces trans-dimerization reaction between the two aromatic ring residues in its molecule and dissociation reaction thereof within the ultraviolet region, the two reactions proceeding in different wavelength regions, (1), and applying the solution to a substrate and then removing the solvent to incorporate the photosensitive compound in the polymeric compound layer.

The present invention also provides photosensitive members or elements comprising a substrate, which transmits light having a wavelength of 300 nm or more, and a photosensitive compound expressed by the formula (I) which is coated on the front and/or the rear side of the substrate. These members are produced by a method comprising the steps of coating on a substrate, which transmits light having a wavelength of 300 nm or more, a solution of a photosensitive compound, which exhibits trans-dimerization between the two aromatic ring residues in its molecule and dissociation of the dimer produced, which proceed in different wavelength regions in the ultraviolet region; and removing the solvent to form a photosensitive layer on the substrate.

The present invention also provides optical circuits comprising an optical path which allows light having a wavelength of 300 nm or more to pass therethrough and an optical switch section composed of a photosensitive compound expressed by the formula |I|.

The present invention also provides optical switches comprising a supporting member which transmits light having a wavelength of 300 nm or more and a photosensitive compound expressed by the formula III which is supported by the supporting member.

The present invention also provides optical logical elements comprising a supporting member which transmits light having a wavelength of 300 nm or more and a photosensitive compound expressed by the formula |I| which is supported by the supporting member.

The present invention also provides optical shutters disposed in an optical path for light having a wavelength of 300 nm or more and comprising a base material which transmits light having a wavelength of 300 nm or more and a photosensitive compound expressed by the formula III which is supported by the supporting member.

The present invention also provides optical filters disposed in an optical path for light with a wavelength of 300 nm or more and comprising a substrate having transmits light having a wavelength of 300 nm or more and a photosensitive compound expressed by the formula III which is supported by the substrate.

The present invention also provides an optical recording medium comprising a substrate which transmits light having a wavelength of 300 nm or more and a photosensitive compound which is expressed by the formula |I| and supported by the substrate.

The present invention also provides optical memory elements comprising a substrate which transmits light having a wavelength of 300 nm or more and a photosensitive compound which is expressed by the formula III and supported by the substrate.

The compounds of formula III of the present invention undergo intramolecular trans-dimerization reaction when irradiated with ultraviolet light having a wavelength of 340 to 380 nm. This reaction is caused by dimerization between the terminal residues (for example, anthracene) of the compounds, resulting in a reduction in length of the resonance structure. This dimerization is simply dissociated by irradiation with ultraviolet light having a wavelength of 240 to 280 nm, thereby returning the compound to the original non- dimerized state. Such reversible photoisomerization is hardly affected by heat or visible light only.

The photosensitive elements such as reversible optical recording media or the like which employ the abovementioned function exhibit an extremely low degree to which a written record is erased by heat or visible light, or recording errors occur, as compared with conventional recording members.

The use of the aforementioned function enables the development of reversible recording materials, optical computer elements such as optical memory elements, optical circuits, optical switches or optical logical elements, very high speed optical shutters or optical filters, which all exhibit excellent repeatability, recording speed and recording stability.

Namely, an optical shutter or an optical switch for cutting off only light having a wavelength of 300 to 400 nm can be obtained by using one of the photosensitive compounds of the present invention. The transmittance can be controlled by using light of 360 nm and 260 nm, with no danger that the transmittance is changed by visible light. There is also no danger of generating highly toxic gas even if a material composed of a compound of the present invention is burnt because the compound used is an organic compound.

The compounds expressed by the formula III of the present invention are also characterized in that they can be simply synthesized at very low cast. As described in the examples, since the compounds are soluble in organic solvents, they can be easily uniformly dispersed or included in a polymeric compound. In addition, the compounds can be easily formed into films because the solutions of the compounds can be coated.

The compounds expressed by the formula |I| of the present invention can be used in combination with various organic compounds, polymeric materials, organic solvents, inorganic compounds, inorganic polymer materials, inorganic solvents and/or inorganic fillers, or they may contain various additives such as photosensitizers, light absorbers and the like.
Fig. 1 is a drawing in which reversibility of the dimerization reaction produced by irradiation with ultraviolet light in a photochromic compound, which is a photosensitive compound obtained in the present invention, is shown by using the change in absorbance;
Fig. 2 is a drawing which shows the thermal stability of a photochromic compound of the present invention;
Fig. 3 is a drawing which shows the thermal stability of a conventional photochromic compound;
Fig. 4 is a schematic perspective view which shows the principle of the application of a polyvinyl chloride film doped with a photochromic compound of the present invention to an optical shutter or a reversible optical filter for optical fibers;
Fig. 5 is a drawing of the configuration of a concept of an optical circuit which uses a photochromic compound of the present invention;
Fig. 6 is a drawing which shows the repeatability of absorbance examined by repeatedly applying ultraviolet light to a photochromic compound of the present invention;
Fig. 7 is a side view of an optical recording medium provided with a photosensitive compound of the present invention; and
Fig. 8 is a schematic drawing of a configuration which shows the principle of the application of a photosensitive compound of the present invention to an optical switch or an optical logical element.

In the following, the invention will be further explained with reference to examples and the drawings.

### EXAMPLE 1

### Synthesis of 1,8-)bis(9-anthracenecarboxy)-octane

5 g of anthracenecarboxylic acid was dissolved in 100 ml of anhydrous chloroform, and 3 ml of thionyl chloride was then dropwise added to the resultant solution, followed by reflux for 10 hours. The reaction solution was then dried up under reduced pressure and then dissolved in 100 ml of anhydrous chloroform. 2 ml of pyridine and 1.2 ml of 1,8-octanediol were added to the resulting solution, followed by agitation at room temperature for 10 hours. The reaction solution was then washed with 1 N hydrochloric acid in order to remove pyridine, well washed with saturated salt water and then dried ove anhydrous sodium sulfate. The reaction solution was then concentrated and purified by silica gel column chromatography (eluant: dichloromethane) to obtain 3.5 g of slightly yellow 1,8-lbis(9-anthracenecarboxy)-octane shown in Table 1 (yield: 77%).

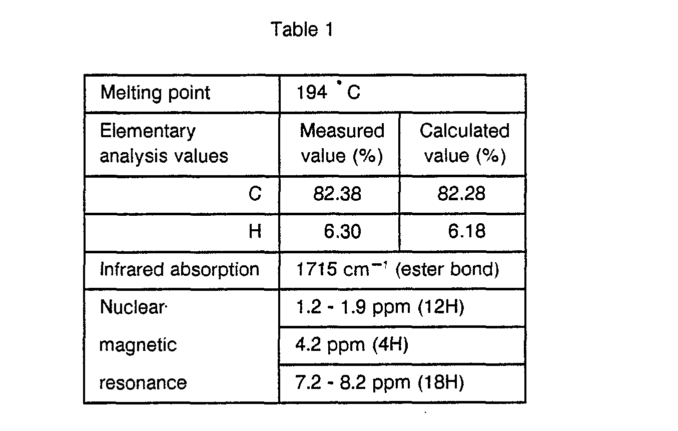

### EXAMPLE 2

### Synthesis of 1,10-ibis(9-anthracenecarboxy)-decane

5 g of anthracene-9-carboxylic acid was dissolved in 100 ml of anhydrous chloroform, and 3 ml of thionyl chloride was then dropwise added to the resultant solution, followed by reflux for 10 hours. The reaction solution was dried up under reduced pressure and then dissolved in 100 ml of anhydrous chloroform. 2 ml of pyridine and 1.4 g of 1,10-decanediol were then added to the resultant solution, followed by agitation at room temperature for 10 hours. The reaction solution was washed with 1 N hydrochloric acid in order to remove pyridine, well washed with saturated salt water and then dried over anhydrous sodium sulfate. The reaction solution was concentrated and then purified by silica gel column chromatography (eluant: dichloromethane) to obtain 3.3 g of the slightly yellow 1,10-lbis(9-anthracenecarboxy)-decane shown in Table 2 (vield: 71 %).

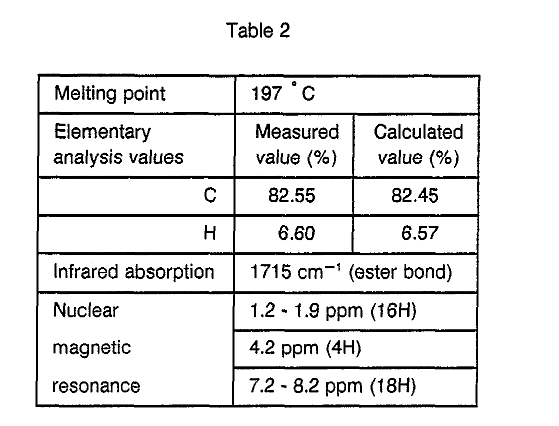

### EXAMPLE 3

### Synthesis of 1,12-jbis(9-anthracenecarboxy)-dodecane

5 g of anthracene-9-carboxylic acid was dissolved in 100 ml of anhydrous chloroform, and 3 ml of thionyl chloride was then dropwise added to the resultant solution, followed by refluxing for 10 hours. The reaction solution was dried up under reduced pressure and then dissolved in 100 ml of anhydrous chloroform. 2 ml of pyridine and 1.7 g of 1,12-dodecanediol were then added to the resultant solution, followed by agitation at room temperature for 10 hours. The reaction solution was washed with 1 N hydrochloric acid in order to remove pyridine, well washed with saturated salt water and then dried over anhydrous sodium sulfate. The reaction solution was concentrated and then purified by silica gel column chromatography (eluant: dichloromethane) to obtain 3.5 g of the slightly yellow 1,12-)bis(9-anthracenecar- boxy)-dodecane shown in Table 3 (yield: 68%).

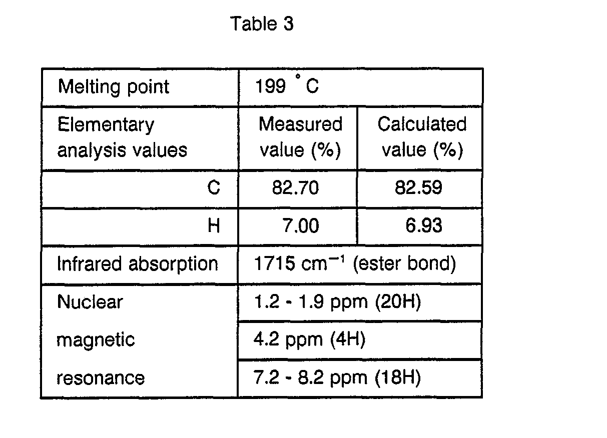

### EXAMPLE 4

### Synthesis of 1,8-|bis(1-anthracenecarboxy)-octane

5 g of anthracene-1-carboxylic acid was dissolved in 100 ml of anhydrous chloroform, and 3 ml of thionyl chloride was then dropwise added to the resultant solution, followed by reflux for 10 hours. The reaction solution was dried up under reduced pressure and then dissolved in 100 ml of anhydrous chloroform. 2 ml of pyridine and 1.2 g of 1,8-octanediol were then added to the resultant solution, followed by agitation at room temperature for 10 hours. The reaction solution was washed with 1 N hydrochloric acid in order to remove pyridine, well washed with saturated salt water and then dried over anhydrous sodium sulfate. The reaction solution was concentrated and then purified by silica gel column chromatography (eluant: dichloromethane) to obtain 3.1 g of the slightly yellow 1,8-lbis(1-anthracenecarboxy)-octane shown in Table 4 (yield: 68%).

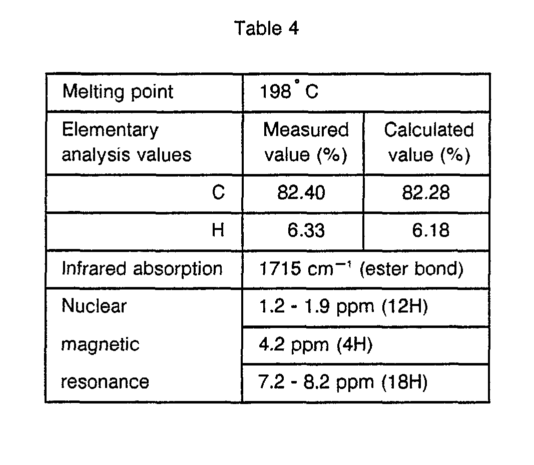

### EXAMPLE 5

### Synthesis of 1,10-lbis(1-anthracenecarboxy)-decane

5 g of anthracene-1-carboxylic acid was dissolved in 100 ml of anhydrous chloroform, and 3 ml of thionyl chloride was then dropwise added to the resultant solution, followed by refluxing for 10 hours. The reaction solution was dried up under reduced pressure and then dissolved in 100 ml of anhydrous chloroform. 2 ml of pyridine and 1.4 g of 1,10-decanediol were then added to the resultant solution, followed by agitation at room temperature for 10 hours. The reaction solution was washed with 1 N hydrochloric acid in order to remove pyridine, well washed with saturated salt water and then dried over anhydrous sodium sulfate. The reaction solution was concentrated and then purified by silica gel column chromatography (eluant: dichloromethane) to obtain 3.3 g of the slightly yellow 1,10-|bis(1-anthracenecarboxy)-decane shown in Table 5 (yield: 71%).

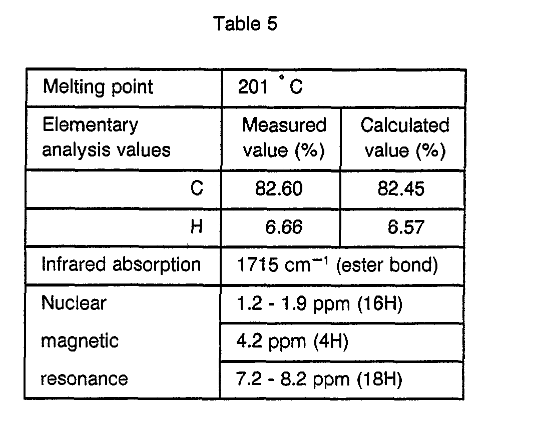

### EXAMPLE 6

### Synthesis of 1,12-|bis(1-anthracenecarboxy)-dodecane

5 g of anthracene-1-carboxylic acid was dissolved in 100 ml of anhydrous chloroform, and 3 ml of thionyl chloride was then dropwise added to the resultant solution, followed by refluxing for 10 hours. The reaction solution was dried up under reduced pressure and then dissolved in 100 ml of anhydrous chloroform. 2 ml of pyridine and 1.7 of 1,12-dodecanediol were then added to the resultant solution, followed by agitation at room temperature for 10 hours. The reaction solution was washed with 1 N hydrochloric acid in order to remove pyridine, well washed with saturated salt water and then dried over anhydrous sodium sulfate. The reaction solution was concentrated and then purified by silica gel column chromatography (eluant: dichloromethane) to obtain 3,3 g of the slightly yellow 1,12-)bis(1-anthracenecar- boxy)-dodecane shown in Table 6 (yield: 64%).

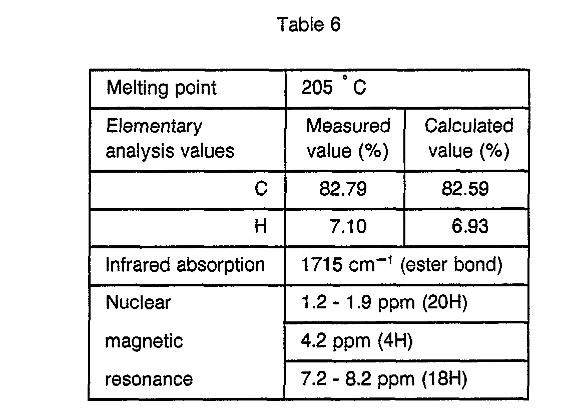

### EXAMPLE 7

### Synthesis of 1,13-|bis(1-anthracenecarboxy)-1,4,7,10,13-pentaoxatridecane

5 g of anthracene-1-carboxylic acid was dissolved in 100 ml of anhydrous chloroform, and 3 ml of thionyl chloride was then dropwise added to the resultant solution, followed by refluxing for 10 hours. The reaction solution was dried up under reduced pressure and then dissolved in 100 ml of anhydrous chloroform. 2 ml of pyridine and 1.6 g of tetraethylene glycol were then added to the resultant solution, followed by agitation at room temperature for 10 hours. The reaction solution was washed with 1 N hydrochloric acid in order to remove pyridine, well washed with saturated salt water and then dried over anhydrous sodium sulfate. The reaction solution was concentrated and then purified by silica gel column chromatography (eluant: dichloromethane) to obtain 3.5 g of the slightly yellow 1,13-lbis(1-anthracenecar- boxy)-1,4,7,10,13-pentaoxatridecane(1,4,7,10,13-pentaoxatridecane-1,13-bis(1-anthracenecarboxylate) shown in Table 7 (yield: 70%).

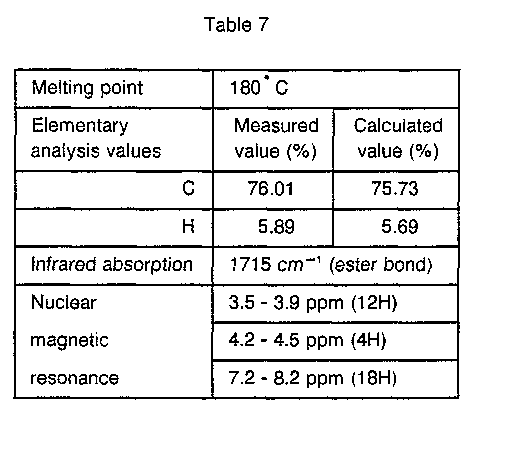

### EXAMPLE 8

### Synthesis of 1,8-lbis(9-anthracenecarbamyl)-octane

5 g of anthracene-9-carboxylic acid was dissolved in 100 ml of anhydrous chloroform, and 3 ml of thionyl chloride was then dropwise added to the resultant solution, followed by reflux for 10 hours. The reaction solution was dried up under reduced pressure and then dissolved in 100 ml of anhydrous chloroform. 2 ml of pyridine and 1.2 g of 1,8-diaminooctane were then added to the resultant solution, followed by agitation at room temperature for 10 hours. After the reaction solution had been filtered, the solid obtained was washed with 1 N hydrochloric acid in order to remove pyridine, well washed with water and methanol in this order and then dried up under reduced pressure. The residue was then purified by silica gel column chromatography (eluant: dichloromethane) to obtain 2.4 g of the slightly yellow 1,8-|bis(9- anthracenecarbamyl)-octane shown in Table 8 (yield: 53%).

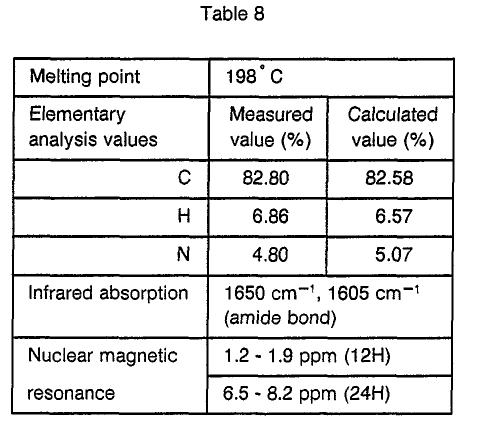

### EXAMPLE 9

### Synthesis of 1,10-|bis(9-anthracenecarbamyl)-decane

5 g of anthracene-9-carboxylic acid was dissolved in 100 ml of anhydrous chloroform, and 3 ml of thionyl chloride was then dropwise added to the resultant solution, followed by reflux for 10 hours. The reaction solution was dried up under reduced pressure and then dissolved in 100 ml of anhydrous chloroform. 2 ml of pyridine and 1.4 g of 1,10-diaminodecane were then added to the resultant solution, followed by agitation at room temperature for 10 hours. After the reaction solution had been filtered, the solid obtained was washed with 1 N hydrochloric acid in order to remove pyridine, well washed with water and methanol in this order and then dried up under reduced pressure. The reside was then purified by silica gel column chromatography (eluant: dichloromethane) to obtain 2.2 g of the slightly yellow 1,10-|bis(9- anthracenecarbamyl)-decane shown in Table 9 (yield: 47%).

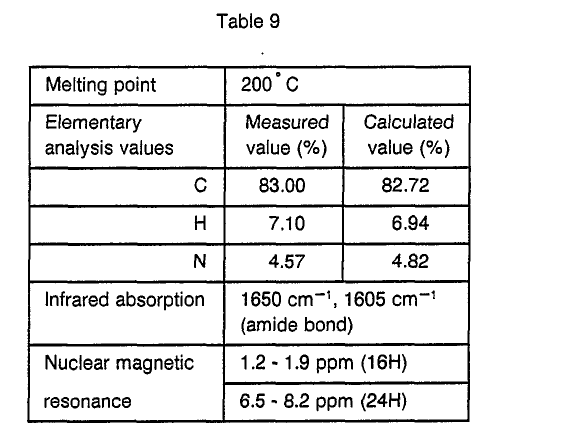

### EXAMPLE 10

### Synthesis of 1,12-|bis(9-anthracenecarbamyl)-dodecane

5 g of anthracene-9-carboxylic acid was dissolved in 100 ml of anhydrous chloroform, and 3 ml of thionyl chloride was then dropwise added to the resultant solution, followed by refluxing for 10 hours. The reaction solution was dried up under reduced pressure and then dissolved in 100 ml of anhydrous chloroform. 2 ml of pyridine and 1.7 g of 1,12-diaminododecane were then added to the resultant solution, followed by agitation at room temperature for 10 hours. After the reaction solution had been filtered, the solid obtained was washed with 1 N hydrochloric acid in order to remove pyridine, well washed with water and methanol in this order and then dried up under reduced pressure. The reside was then purified by silica gel column chromatography (eluant: dichloromethane) to obtain 1.7 g of the slightly yellow 1, 12-lbis(9- anthracenecarbamyl)-dodecane shown in Table 10 (yield: 33%).

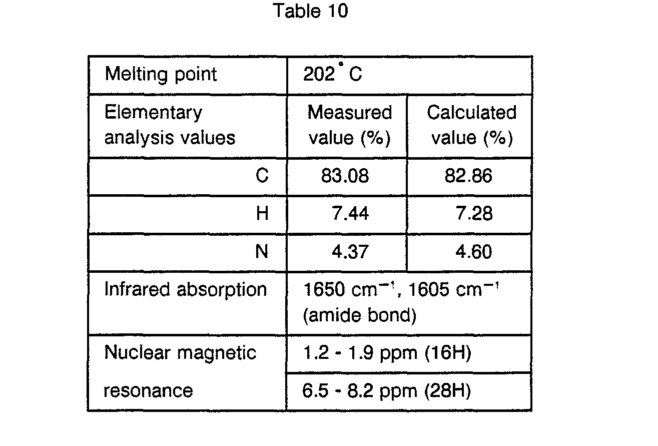

### EXAMPLE 11

### Synthesis of 11-(9-anthracenecarboxy)-8-(1-naphthoxy)-octane

1 g of anthracene-9-carboxylic acid was dissolved in 20 ml of anhydrous chloroform, and 1 ml of thionyl chloride was then dropwise added to the resultant solution, followed by refluxing for 10 hours. The reaction solution was dried up under reduced pressure and then dissolved in 10 ml of anhydrous chloroform. The resultant solution was then dropwise added to a solution obtained by dissolving 1 ml of pyridine and 2 g of 1,8-octanediol in 40 ml of anhydrous chloroform, followed by agitation at room temperature for 10 hours. The reaction solution was washed with 1 N hydrochloric acid in order to remove pyridine, well washed with saturated salt water and then dried over anhydrous sodium sulfate. Chloroform was then removed under reduced pressure, and 100 ml of methanol was then added to the residue to separate a solid. The solid obtained was 1-(9-anthracenecarboxy)-8-octanol.

The well-dried solid of 1-(9-anthracenecarboxy)-8-octanol was dissolved in 30 ml of anhydrous chloroform, and 1 ml of pyridine and 1 g of 1-naphthoyl chloride (naphthyl chloride) were then added to the resultant solution, followed by agitation at room temperature for 10 hours. The reaction solution was then washed with 1 N hydrochloric acid in order to remove pyridine, well washed with saturated salt water and then dried over anhydrous sodium sulfate. The solution was then purified by silica gel column chromatography (eluant: dichloromethane) to obtain 1.2 g of the slightly yellow 1-(9-anthracenecarboxy)-8-(1-naph- thoxy)-octane shown in Table 11 (yield: 53%).

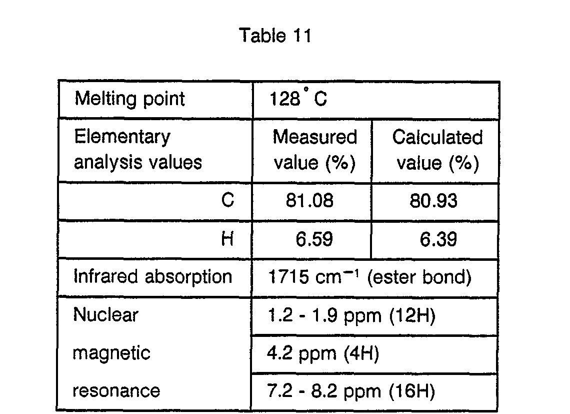

### EXAMPLE 12

### Synthesis of j1-(9-anthracenecarboxy)-10-(1-naphthoxy)-decane

1 g of anthracene-9-carboxylic acid was dissolved in 20 ml of anhydrous chloroform, and 1 ml of thionyl chloride was then dropwise added to the resultant solution, followed by refluxing for 10 hours. The reaction solution was dried up under reduced pressure and then dissolved in 10 ml of anhydrous chloroform. The resultant solution was then dropwise added to a solution obtained by dissolving 1 ml of pyridine and 2.5 g of 1,10-decanediol in 40 ml of anhydrous chloroform, followed by agitation at room temperature for 10 hours. The reaction solution was washed with 1 N hydrochloric acid in order to remove pyridine, well washed with saturated salt water and then dried over anhydrous sodium sulfate. Chloroform was then removed under reduced pressure, and 100 ml of methanol was then added to the residue to separate a solid. The solid obtained was 1-(9-anthracenecarboxy)-10-decanol.

The well-dried solid of 1-(9-anthracenecarboxy)-10-decanol was dissolved in 30 ml of anhydrous chloroform, and 1 ml of pyridine and 1 g of 1-naphthoyl chloride (naphthyl chloride) were then added to the resultant solution, followed by agitation at room temperature for 10 hours. The reaction solution was then washed with 1 N hydrochloric acid in order to remove pyridine, well washed with saturated salt water and then dried over anhydrous sodium sulfate. The solution was then purified by silica gel column chromatography (eluant: dichloromethane) to obtain 1.1 g of the slightly yellow |1-(9-anthracenecarbo×y)-10-(1-naphthoxy)-decane shown in Table 12 (yield: 46%).

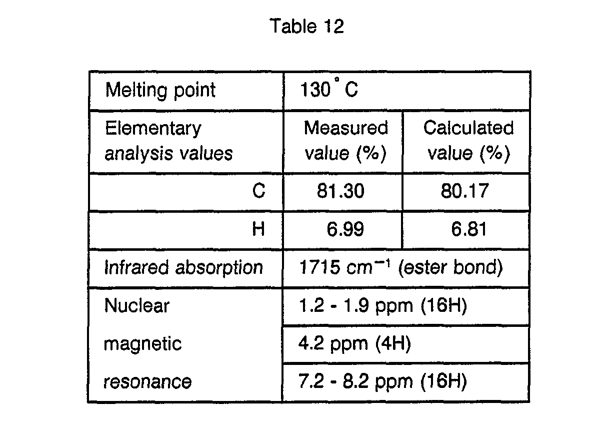

### EXAMPLE 13

### Synthesis of j1-(9-anthracenecarboxy)-12-(1-naphthoxy)-dodecane

1 g of anthracene-9-carboxylic acid was dissolved in 20 ml of anhydrous chloroform, and 1 ml of thionyl chloride was then dropwise added to the resultant solution, followed by refluxing for 10 hours. The reaction solution was dried up under reduced pressure and then dissolved in 10 ml of anhydrous chloroform. The resultant solution was then dropwise added to a solution obtained by dissolving 1 ml of pyridine and 3 of 1,12-dodecanediol in 40 ml of anhydrous chloroform, followed by agitation at room temperature for 10 hours. The reaction solution was washed with 1 N hydrochloric acid in order to remove pyridine, well washed with saturated salt water and then dried over anhydrous sodium sulfate. Chloroform was then removed under reduced pressure, and 100 ml of methanol was then added to the residue to separate a solid. The solid obtained was 1-(9-anthracenecarboxy)-12-dodecanol.

The well-dried solid of 1-(9-anthracenecarboxy)-12-dodecanol was dissolved in 30 ml of anhydrous chloroform, and 1 ml of pyridine and 1 g of 1-naphthoyl chloride were then added to the resultant solution, followed by agitation at room temperature for 10 hours. The reaction solution was then washed with 1 N hydrochloric acid in order to remove pyridine, well washed with saturated salt water and then dried over anhydrous sodium sulfate. The solution was then purified by silica gel column chromatography (eluant: dichloromethane) to obtain 0.9 g of the slightly yellow 11-(9-anthracenecarboxy)-12-(1-naphthoxy)-dodecane shown in Table 13 (yield: 36%).

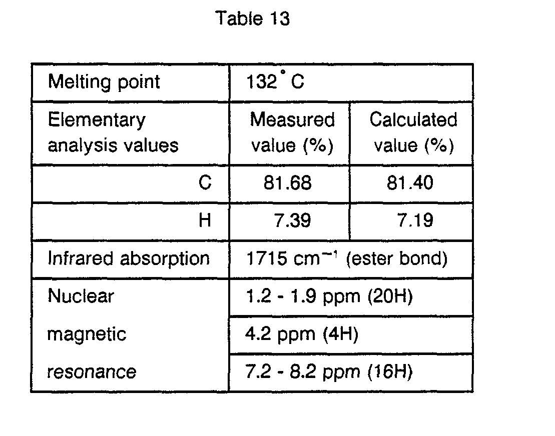

### EXAMPLE 14

### Synthesis of j1-(9-anthracenecarboxy)-8-(2-naphthoxy)-octane

1 g of anthracene-9-carboxylic acid was dissolved in 20 ml of anhydrous chloroform, and 1 ml of thionyl chloride was then dropwise added to the resultant solution, followed by refluxing for 10 hours. The reaction solution was dried up under reduced pressure and then dissolved in 10 ml of anhydrous chloroform. The resultant solution was then dropwise added to a solution obtained by dissolving 1 ml of pyridine and 3 g of 1,8-octanediol in 40 ml of anhydrous chloroform, followed by agitation at room temperature for 10 hours. The reaction solution was washed with 1 N hydrochloric acid in order to remove pyridine, well washed with saturated salt water and then dried over anhydrous sodium sulfate. Chloroform was then removed under reduced pressure, and 100 ml of methanol was then added to the residue to separate a solid. The solid obtained was 1-(9-anthracenecarboxy)-8-octanol.

The well-dried solid of 1-(9-anthracenecarboxy)-8-octanol was dissolved in 30 ml of anhydrous chloroform, and 1 ml of pyridine and 1 g of 2-naphthoyl chloride were then added to the resultant solution, followed by agitation at room temperature for 10 hours. The reaction solution was then washed with 1 N hydrochloric acid in order to remove pyridine, well washed with saturated salt water and then dried over anhydrous sodium sulfate. The solution was then purified by silica gel column chromatography (eluant: dichloromethane) to obtain 1.5 g of the slightly yellow [1-(9-anthracenecarboxy)-8-(2-naphthoxy)-octane shown in Table 14 (yield: 66%).

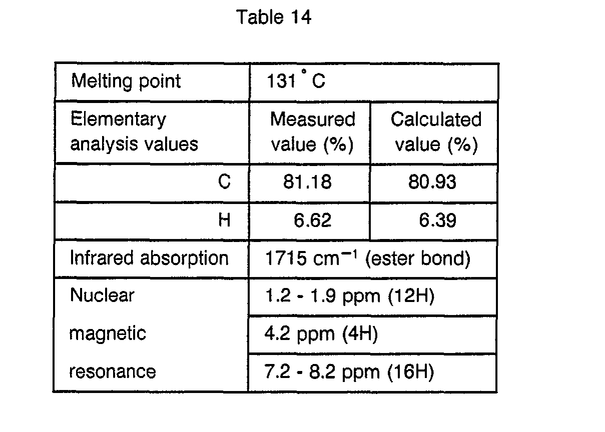

### EXAMPLE 15

### Synthesis of |1-(9-anthracenecarboxy)-10-(2-naphthoxy)-decane

1 g of anthracene-9-carboxylic acid was dissolved in 20 ml of anhydrous chloroform, and 1 ml of thionyl chloride was then dropwise added to the resultant solution, followed by refluxing for 10 hours. The reaction solution was dried up under reduced pressure and then dissolved in 10 ml of anhydrous chloroform. The resultant solution was then dropwise added to a solution obtained by dissolving 1 ml of pyridine and 2.5 g of 1,10-decanediol in 40 ml of anhydrous chloroform, followed by agitation at room temperature for 10 hours. The reaction solution was washed with 1 N hydrochloric acid in order to remove pyridine, well washed with saturated salt water and then dried over anhydrous sodium sulfate. Chloroform was then removed under reduced pressure, and 100 ml of methanol was then added to the residue to separate a solid. The solid obtained was 1-(9-anthracenecarboxy)-10-decanol.

The well-dried solid of 1-(9-anthracenecarboxy)-10-decanol was dissolved in 30 ml of anhydrous chloroform, and 1 ml of pyridine and 1 g of 2-naphthoyl chloride were then added to the resultant solution, followed by agitation at room temperature for 10 hours. The reaction solution was then washed with 1 N hydrochloric acid in order to remove pyridine, well washed with saturated salt water and then dried over anhydrous sodium sulfate. The solution was then purified by silica gel column chromatography (eluant: dichloromethane) to obtain 1.4 g of the slightly yellow ll-(9-anthracenecarboxy)-10-(2-naphthoxy)-decane shown in Table 15 (yield: 58%).

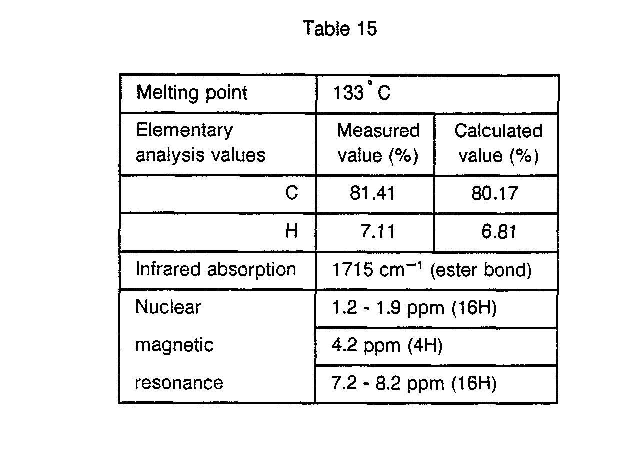

### EXAMPLE 16

### Synthesis of !1-(9-anthracenecarboxy)-12-(2-naphthoxy)-dodecane

1 g of anthracene-9-carboxylic acid was dissolved in 20 ml of anhydrous chloroform, and 1 ml of thionyl chloride was then dropwise added to the resultant solution, followed by refluxing for 10 hours. The reaction solution was dried up under reduced pressure and then dissolved in 10 ml of anhydrous chloroform. The resultant solution was then dropwise added to a solution obtained by dissolving 1 ml of pyridine and 3 g of 1,12-dodecanediol in 40 ml of anhydrous chloroform, followed by agitation at room temperature for 10 hours. The reaction solution was washed with 1 N hydrochloric acid in order to remove pyridine, well washed with saturated salt water and then dried over anhydrous sodium sulfate. Chloroform was then removed under reduced pressure, and 100 ml of methanol was then added to the residue to separate a solid. The solid obtained was 1-(9-anthracenecarboxy)-12-dodecanol.

The well-dried solid of 1-(9-anthracenecarboxy)-12-dodecanol was dissolved in 30 ml of anhydrous chloroform, and 1 ml of pyridine and 1 g of 2-naphthoyl chloride were then added to the resultant solution, followed by agitation at room temperature for 10 hours. The reaction solution was then washed with 1 N hydrochloric acid in order to remove pyridine, well washed with saturated salt water and then dried over anhydrous sodium sulfate. The solution was then purified by silica gel column chromatography (eluant: dichloromethane) to obtain 1.2 g of the slightly yellow 11-(9-anthracenecarboxy)-12-(2-naphthoxy)-dodecane shown in Table 16 (yield: 48%).

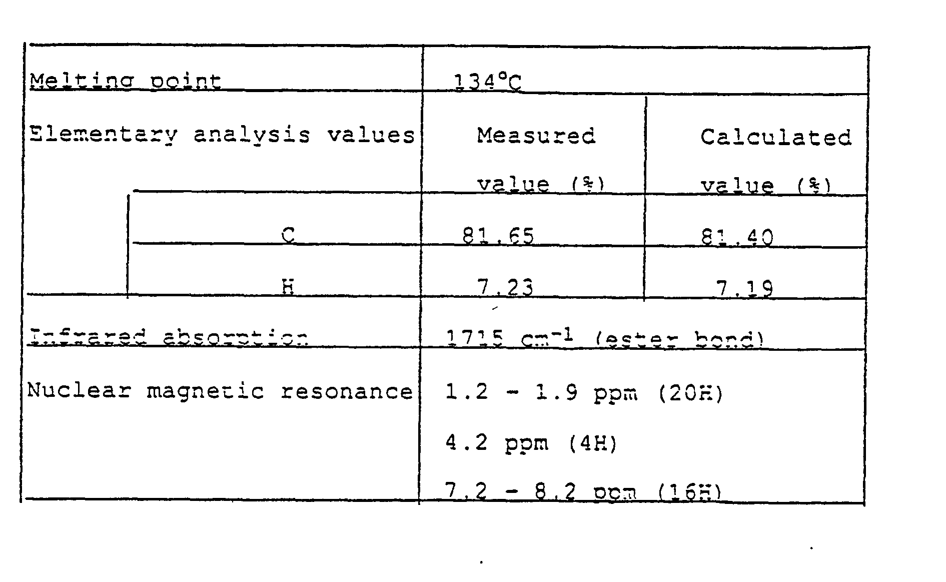

### EXAMPLE 17

### Synthesis of 1-(1-anthracenecarboxy)-8-(1-naphthoxy)-octane

1 g of anthracene-1-carboxylic acid was dissolved in 20 ml of anhydrous chloroform, and 1 ml of thionyl chloride was then dropwise added to the resultant solution, followed by refluxing for 10 hours. The reaction solution was dried up under reduced pressure and then dissolved in 10 ml of anhydrous chloroform. The resultant solution was then dropwise added to a solution obtained by dissolving 1 ml of pyridine and 2 g of 1,8-octanediol in 40 ml of anhydrous chloroform, followed by agitation at room temperature for 10 hours. The reaction solution was washed with 1 N hydrochloric acid in order to remove pyridine, well washed with saturated salt water and then dried over anhydrous sodium sulfate. Chloroform was then removed under reduced pressure, and 100 ml of methanol was then added to the residue to separate a solid. The solid obtained was 1-(1-anthracenecarboxy)-8-octanol.

The well-dried solid of 1-(1-anthracenecarboxy)-8-octanol was dissolved in 30 ml of anhydrous chloroform, and 1 ml of pyridine and 1 g of 1-naphthoyl chloride were then added to the resultant solution, followed by agitation at room temperature for 10 hours. The reaction solution was then washed with 1 N hydrochloric acid in order to remove pyridine, well washed with saturated salt water and then dried over anhydrous sodium sulfate. The solution was then purified by silica gel column chromatography (eluant: dichloromethane) to obtain 1.4 g of the slightly yellow 11-(1-anthracenecarboxy)-8-(1-naphthoxy)-octane shown in Table 17 (yield: 62%).

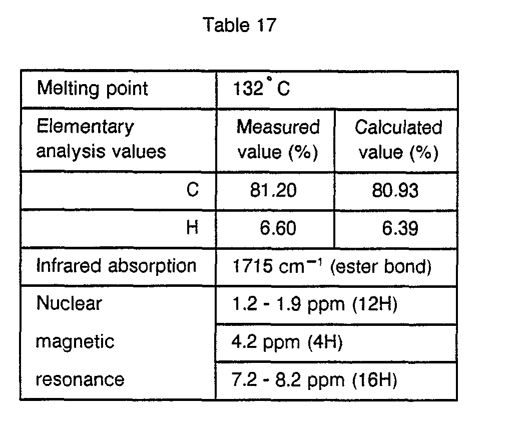

### EXAMPLE 18

### Synthesis of )1-(1-anthracenecarboxy)-10-(1-naphthoxy)-decane

1 g of anthracene-1-carboxylic acid was dissolved in 20 ml of anhydrous chloroform, and 1 ml of thionyl chloride was then dropwise added to the resultant solution, followed by refluxing for 10 hours. The reaction solution was dried up under reduced pressure and then dissolved in 10 ml of anhydrous chloroform. The resultant solution was then dropwise added to a solution obtained by dissolving 1 ml of pyridine and 2.5 g of 1,10-decanediol in 40 ml of anhydrous chloroform, followed by agitation at room temperature for 10 hours. The reaction solution was washed with 1 N hydrochloric acid in order to remove pyridine, well washed with saturated salt water and then dried over anhydrous sodium sulfate. Chloroform was then removed under reduced pressure, and 100 ml of methanol was then added to the residue to separate a solid. The solid obtained was 1-(1-anthracenecarboxy)-10-decanol.

The well-dried solid of 1-(1-anthracenecarboxy)-10-decanol was dissolved in 30 ml of anhydrous chloroform, and 1 ml of pyridine and 1 g of 1-naphthoyl chloride were then added to the resultant solution, followed by agitation at room temperature for 10 hours. The reaction solution was then washed with 1 N hydrochloric acid in order to remove pyridine, well washed with saturated salt water and then dried over anhydrous sodium sulfate. The solution was then purified by silica gel column chromatography (eluant: dichloromethane) to obtain 1.4 g of the slightly yellow |1-(1-anthracenecarboxy)-10-(1-naphthoxy)-decane shown in Table 18 (yield: 58%).

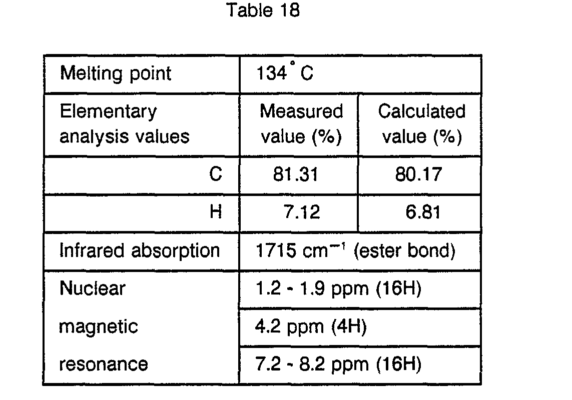

### EXAMPLE 19

### Synthesis of 11-(1-anthracenecarboxy)-12-(1-naphthoxy)-dodecane

1 g of anthracene-1-carboxylic acid was dissolved in 20 ml of anhydrous chloroform, and 1 ml of thionyl chloride was then dropwise added to the resultant solution, followed by reflux for 10 hours. The reaction solution was dried up under reduced pressure and then dissolved in 10 ml of anhydrous chloroform. The resultant solution was then dropwise added to a solution obtained by dissolving 1 ml of pyridine and 3 g of 1,12-decanediol in 40 ml of anhydrous chloroform, followed by agitation at room temperature for 10 hours. The reaction solution was washed with 1 N hydrochloric acid in order to remove pyridine, well washed with saturated salt water and then dried over anhydrous sodium sulfate. Chloroform was then removed under reduced pressure, and 100 ml of methanol was then added to the residue to separate a solid. The solid obtained was 1-(1-anthracenecarboxy)-12-dodecanol.

The well-dried solid of 1-(1-anthracenecarboxy)-12-dodecanol was dissolved in 30 ml of anhydrous chloroform, and 1 ml of pyridine and 1 g of 1-naphthoyl chloride were then added to the resultant solution, followed by agitation at room temperature for 10 hours. The reaction solution was then washed with 1 N hydrochloric acid in order to remove pyridine, well washed with saturated salt water and then dried over anhydrous sodium sulfate. The solution was then purified by silica gel column chromatography (eluant: dichloromethane) to obtain 1.1 g of the slightly yellow 11-(1-anthracenecarboxy)-12-(1-naphthoxy)-dodecane shown in Table 19 (yield: 44%).

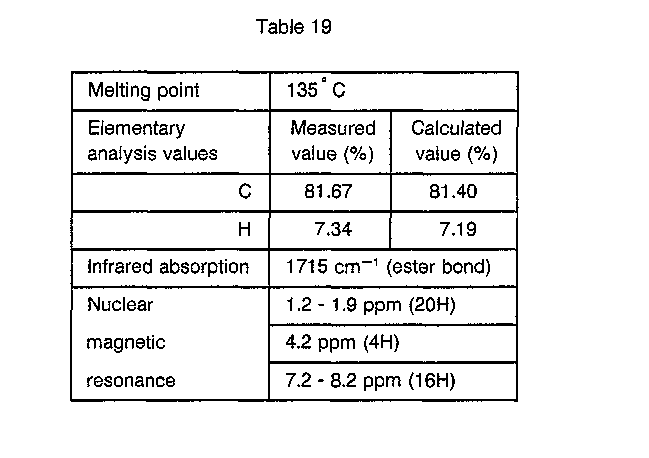

### EXAMPLE 20

### Synthesis of ]1-(1-anthracenecarboxy)-1 0-(1-naphthylsulfonyloxy)-decane

1 g of anthracene-1-carboxylic acid was dissolved in 20 ml of anhydrous chloroform, and 1 ml of thionyl chloride was then dropwise added to the resultant solution, followed by reflux for 10 hours. The reaction solution was dried up under reduced pressure and then dissolved in 10 ml of anhydrous chloroform. The resultant solution was then dropwise added to a solution obtained by dissolving 1 ml of pyridine and 3 g of 1,10-decanediol in 40 ml of anhydrous chloroform, followed by agitation at room temperature for 10 hours. The reaction solution was washed with 1 N hydrochloric acid in order to remove pyridine, well washed with saturated salt water and then dried over anhydrous sodium sulfate. Chloroform was then removed under reduced pressure, and 100 ml of methanol was then added to the residue to separate a solid. The solid obtained was 1-(1-anthracenecarboxy)-10-decanol.

The well-dried solid of 1-(1-anthracenecarboxy)-10-decanol was dissolved in 30 ml of anhydrous chloroform, and 1 ml of pyridine and 1 g of 1-naphthalenesulfonyl chloride were then added to the resultant solution, followed by agitation at room temperature for 10 hours. The reaction solution was then washed with 1 N hydrochloric acid in order to remove pyridine, well washed with saturated salt water and then dried over anhydrous sodium sulfate. The solution was then purified by silica gel column chromatography (eluant: dichloromethane) to obtain 1.1 g of the slightly yellow |1-(1-anthracenecarboxy)-10-(1-naphthylsulfonyloxy)-decane shown in Table 20 (yield: 44%).

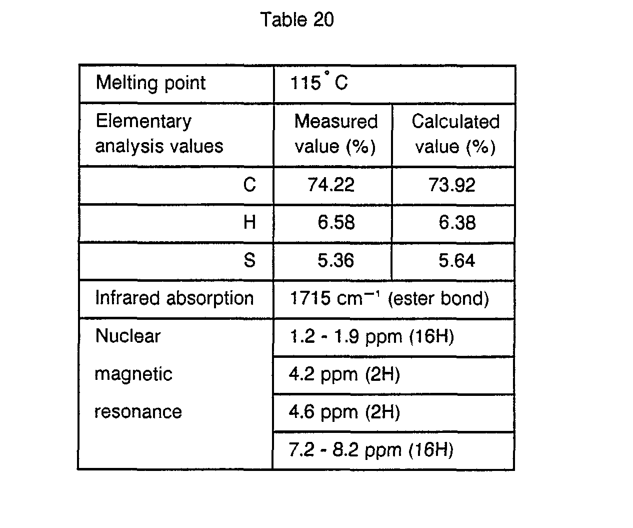

### EXAMPLE 21

### Synthesis of |1-(1-anthracenecarbonamido)-10-(1-naphthylsulfonylamino)-decane

1 g of anthracene-1-carboxylic acid was dissolved in 20 ml of anhydrous chloroform, and 1 ml of thionyl chloride was then dropwise added to the resultant solution, followed by refluxing for 10 hours. The reaction solution was dried up under reduced pressure and then dissolved in 10 ml of anhydrous chloroform. The resultant solution was then dropwise added to a solution obtained by dissolving 2.5 g of 1.10-diaminodecane in 40 ml of anhydrous chloroform, followed by agitation at room temperature for 10 hours. After the reaction solution had been filtered, the solid obtained was washed with 1 N hydrochloric acid in order to remove excess 1,10-diaminodecane, well washed with water and methanol in this order and then dried under reduced pressure. The thus-obtained solid was 1-(1-anthracenecarbamyl)-10-decaneamine(1-(1-anth- racenecarbonamido)-10-aminodecane).

The well-dried solid of 1-(1-anthracenecarbamyl)-10-decaneamine was dissolved in 30 ml of anhydrous pyridine, and 1 g of 1-naphthalenesulfonyl chloride was then added to the resultant solution, followed by agitation at room temperature for 10 hours. After the reaction solution had been filtered, the solid obtained was washed with 1 N hydrochloric acid in order to remove pyridine, well washed with water and methanol in this order, and then dried under reduced pressure. The product was then purified by silica gel column chromatography (eluant: dichloromethane) to obtain 1.1 g of the slightly yellow p-(1-anthracenecarbamyl)-10-(1-naphthylsulfonylamino)-decane(1-(1-anthracenecarbonamido)-10-(1-naphthylsulfonylamino)-decane) shown in Table 21 (yield: 43%).

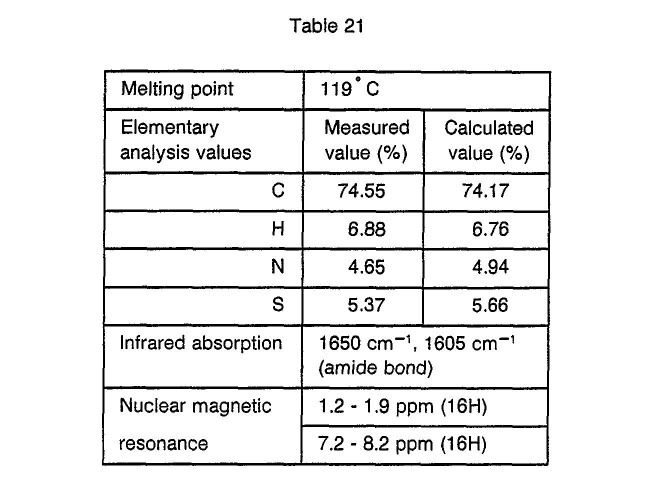

### EXAMPLE 22;

### Synthesis of 1,10-lbis(9-anthracenemethoxy)-decane

5 g of 9-hydroxymethylanthracene was dissolved in 20 ml of acetone, and 2.5 g of KHCO₃ was then added to the resultant solution, followed by refluxing. After 5 minutes, a solution obtained by dissolving 3 g of 1,10-dibromodecane in 20 ml of acetone was dropwise added to the resultant solution, followed by refluxing for 5 hours. After the reaction had been completed, the reaction solution was cooled to room temperature and the filtered in order to remove KHCO₃. The filtrate was dried up under reduced pressure and then purified by silica gel column chromatography (eluant: chloroform) to obtain 2 g of the slightly yellow 1,10-|bis(9-anthracenemethoxy)-decane shown in Table 22 (yield: 36%).

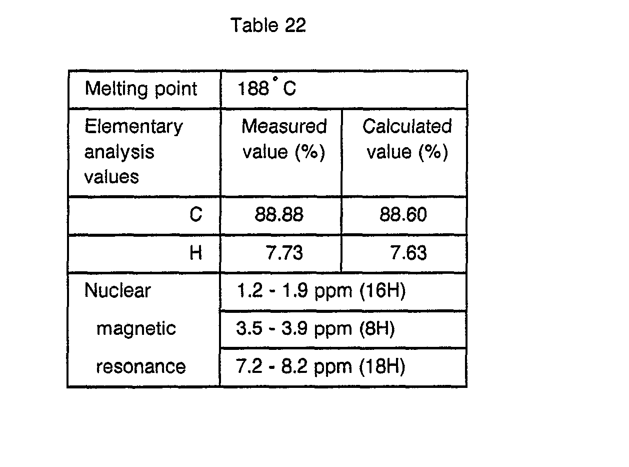

### EXAMPLE 23

### Synthesis of 1,10-|bis(9-anthraceneamino)decane

3 g of 9-aminoanthracene was dissolved in 50 ml of ethanol, and 2.5 g of K₂COa was then added to the resultant solution, followed by refluxing. After 5 minutes a solution obtained by dissolving 2 g of 1,10-dibromodecane in 20 ml of ethanol was dropwise added to the resultant solution, followed by refluxing for 5 hours. After reaction had been completed, the reaction solution was cooled to room temperature and then filtered in order to remove K₂CO₃. The filtrate was dried up under reduced pressure and then purified by neutral alumina column chromatography (eluant: ether : hexane = 1 : 3) to obtain 1.6 g of the slightly yellow 1,10-lbis(9-anthraceneamino)-decane shown in Table 23 (yield: 45%).

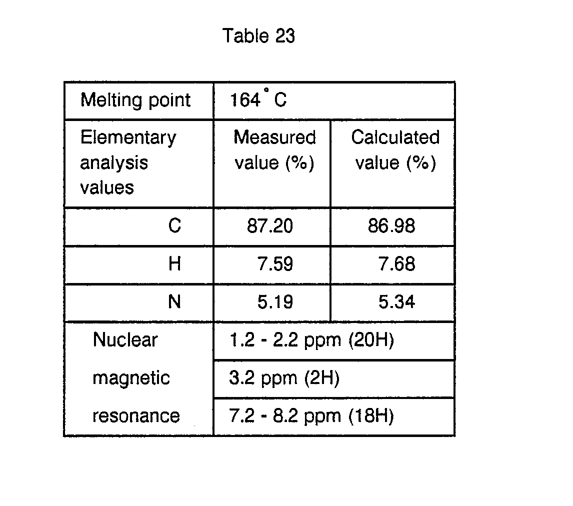

### EXAMPLE 24

### Synthesis of 1,10-lbis(9-anthracene)-decane

2.8 g of anhydrous aluminum chloride was dissolved in 60 ml of chloroform, and a solution obtained by dissolving 3 g of 1,10-dibromodecane in 20 ml chloroform was then dropwise added to the resultant solution, followed by refluxing for 10 minutes. A solution obtained by dissolving 5 g of anthracene in 20 ml of ethanol was dropwise added to the resultant solution over a time of 1 hour, followed by refluxing for 1 hour. After reaction had been completed, the reaction solution was cooled to room temperature and then dropwise added to 200 ml of an aqueous solution of 10% hydrochloric acid. The chloroform phase was separated, well washed with saturated salt water and then dried over anhydrous sodium sulfate. The thus-obtained solution was concentrated and then purified by silica gel column chromatography (eluant: chloroform) to obtain 1.8 g of the slightly yellow 1,10-ibis(9-anthracene)-decane shown in Table 24 (yield: 36%).

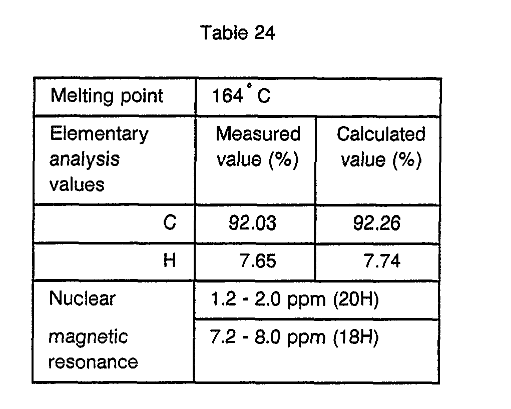

### EXAMPLE 25

### Synthesis of 1,4-(bis(9-anthracenecarboxy)-benzene

3 g of 9-hydroxymethylanthracene was dissolved in 100 ml of anhydrous chloroform, and 2 ml of pyridine and 1 g of terephthalic acid chloride were then added to the resultant solution, followed by refluxing for 10 hours. After reaction had been completed, the reaction solution was cooled to room temperature, washed with 1 N hydrochloric acid in order to remove pyridine, well washed with saturated salt water and then dried over anhydrous sodium sulfate. The thus-obtained solution was purified by silica gel column chromatography (eluant: dichloromethane) to obtain 1.4 g of the yellow 1,4-|bis(9-anthracenecar- boxy)-benzene shown in Table 25 (yield: 52%).

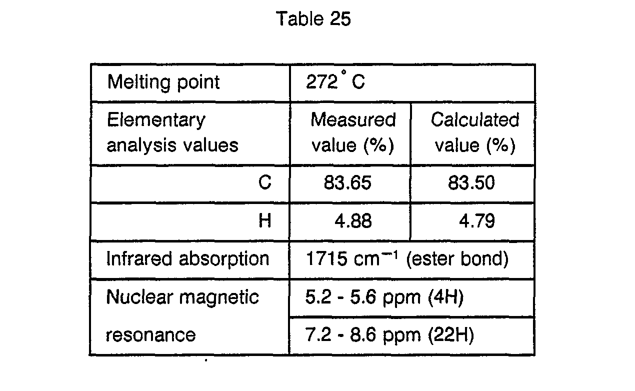

### EXAMPLE 26

The absorbance change caused by irradiation with light was examined by using a solution of one of the compounds synthesized in the present examples.

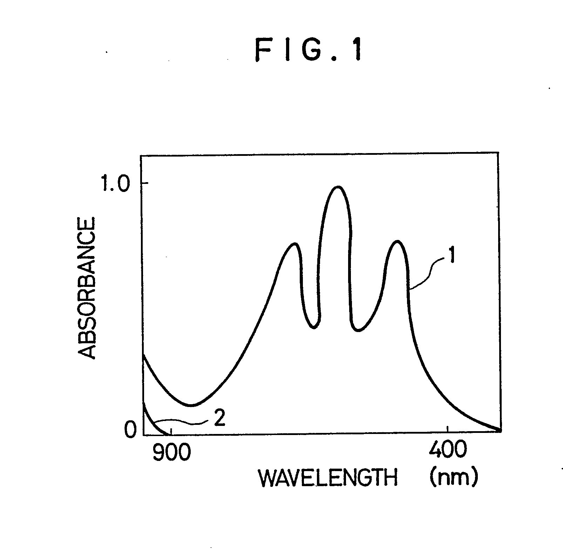

Fig. 1 shows the absorption band of a 5 x 10-⁵ mol/I dichloromethane solution of 1,8-|bis(9- anthracenecarboxy)-octane synthesized in EXAMPLE 1. The absorption (the curve 1 shown in Fig. 1) arising from anthracene is shown in the region of 300 to 420 nm.

This absorption band completely disappeared (curve 2 shown in Fig. 1) when the dichloromethane solution was irradiated with near ultraviolet light at 360 nm for 3 minutes by using a 500-W xenon lamp as a light source.

The original absorption band (curve 1 shown in Fig. 1) appeared again when the dichloromethane solution was irradiated with ultraviolet light at 260 nm for 3 minutes by using a 500-W xenon lamp as a light source. This result revealed the fact that this photochemical reaction is reversible.

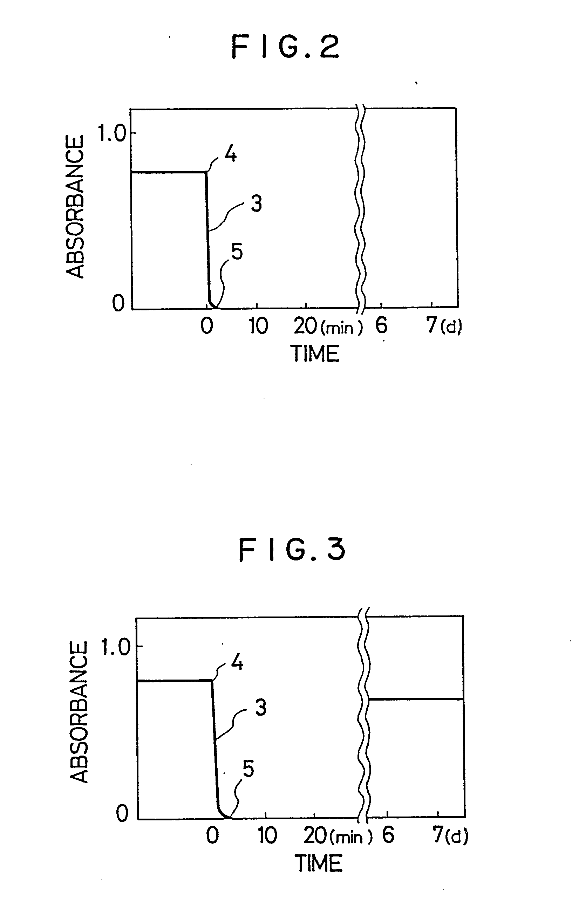

Fig. 2 shows the change of the absorbance with time at a wavelength of 380 nm.

For the purpose of comparison, Fig. 3 shows the change of the absorbance with time of 1,4-|bis(9- anthracenecarboxy)-butane at 380 nm which produces a cis-type optical dimerization reaction. The absorbance of either of the compounds was reduced by irradiation with near ultraviolet light at 360 nm, as shown by the downward curve 3. The upper end 4 and the lower end 5 of the downward curve 3 indicate the start point and the end point, respectively, of the irradiation with near ultraviolet light at 360 nm. When both compounds were then allowed to stand in a dark place, 1,8-lbis-(9-anthracenecarboxy)-octane of the present invention showed no change in the absorbance 7 days after the irradiation with near ultraviolet light at 360 nm, as shown in Fig. 2, while 1,4-lbis(9-anthracenecarboxy)-butane of a comparative example showed a change in the absorbance in an early stage after it had been allowed to stand for 10 to 30 minutes after the irradiation with near ultraviolet light at 360 nm and a great change 6 days after the irradiation, as shown in Fig. 3.

This reveals the fact that the photochromic compound of the present invention is superior to a conventional photochromic compound with respect to the thermal stability with time.

### EXAMPLE 27

A 5 x 10-⁵ molll dichloromethane solution of 1,12-ibis(9-anthracenecarboxy)-dodecane synthesized in EXAMPELE 3 showed the absorption in the wavelength range of 300 to 420 nm, which arises from anthracene. This absorption band completely disappeared when the solution was irradiated with near ultraviolet light at 360 nm. The absorption band which disappeared was not at all recovered even if the solution was allowed to stand under visible light. However, the original absorption band appeared when the solution was irradiated with ultraviolet light at 260 nm. This makes it apparent that the photochemical reaction is reversible.

### EXAMPLE 28

A dichloromethane solution of 5 x 10-⁵ mol/I of 1,12-lbis(l-anthracenecarboxy)-dodecane synthesized in EXAMPLE 6 showed absorption within the range of 300 to 420 nm, which arises from anthracene. This absorption band completely disappeared when the solution was irradiated with near ultraviolet light at 360 nm. The disappeared band was not at all recovered even if the solution was allowed to stand under visible light. However, the original absorption band appeared when the solution was irradiated with ultraviolet light at 260 nm. This revealed that fact that this photochemical reaction is reversible.

### EXAMPLE 29

A dichloromethane solution of 5 x 10-⁵ mol/l of |1-(9-anthracenecarboxy)-12-(1-naphthoxy)-dodecane synthesized in EXAMPLE 13 showed the absorption within the range of 300 to 420 nm, which arises from anthracene. This absorption band completely disappeared when the solution ws irradiated with near ultraviolet light at 360 nm. The disappeared band was not at all recovered even if the solution was allowed to stand under visible light. However, the original absorption band appeared when the solution was irradiated with ultraviolet light at 260 nm. This revealed the fact that this photochemical reaction is reversible.

### EXAMPLE 30

The change in absorbance was examined by applying light to a polymer film composed of a photochromic compound of the present invention.

8.5 mg of 1,8-lbis(9-anthracenecarboxy)-octane synthesized in EXAMPLE 1 and 1 g of polyvinyl chloride, which transmits light at a wavelength of 300 nm or more, were dissolved in 10 ml of tetrahydrofuran. The resultant solution was then poured into a petri dish having an inner diameter of 6 cm and then dried at room temperature to form a film having a thickness of 0.24 mm.

As a result of measurement of the absorbance of this film, the film showed a reabsorption band within the range of 300 to 420 nm. After this film had been irradiated with near ultraviolet light at 360 nm, the absorbance of the film was again measured As a result, the absorption band arising from anthracene completely disappeared. After the film had been allowed to stand under visible light for 7 days, the absorbance was again measured. As a result, the absorption band remained disappeared. The absorbance was thus measured after the film had been irradiated with ultraviolet light at 260 nm. As a result, the original absorption band arising from anthracene appeared. Such a property of this film enables the film to be used as an optical memory element.

### EXAMPLE 31

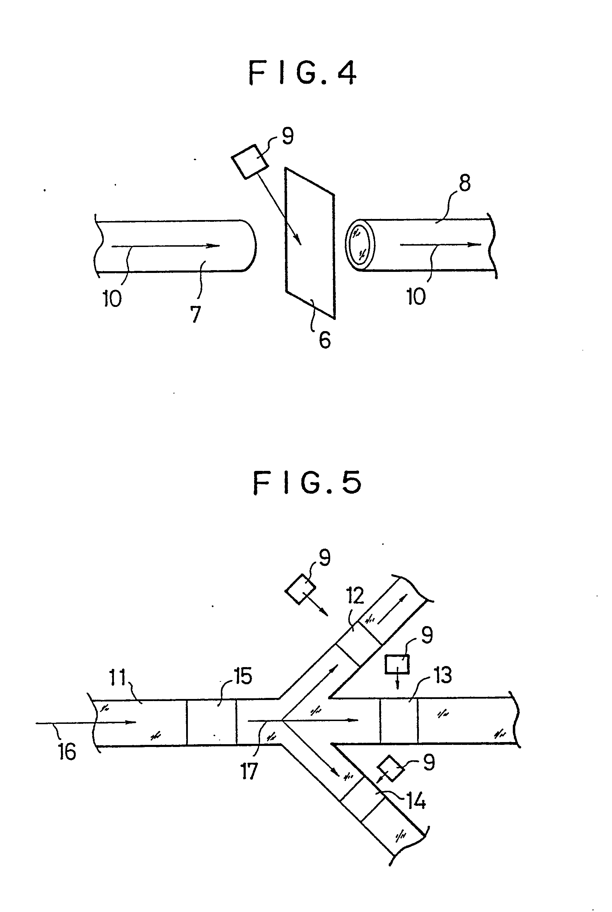

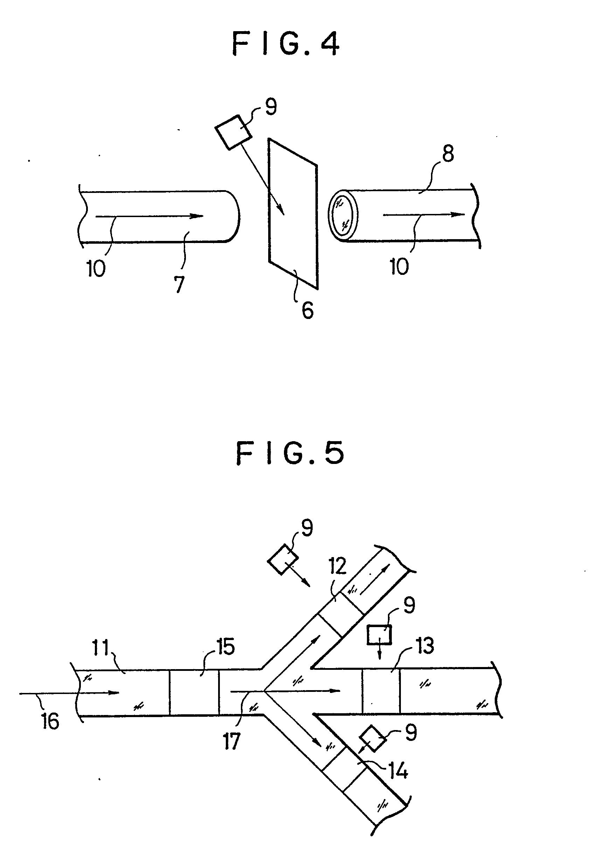

The polyvinyl chloride film 6 doped with 1,8-lbis(9-anthracenecarboxy)-octane which was formed in EXAMPLE 30 was placed between optical fibers 7 and 8, as shown in Fig. 4. This film hardly transmits light having a wavelength of 390 nm which is half of the wavelength of 780 nm of laser light. However, this film had a transmission of about 100% for the light 10 at 390 nm when this film was irradiated with near ultraviolet light at 360 nm by using an irradiation apparatus 9. When the film was then irradiated with ultraviolet light at 260 nm by using the irradiation apparatus 9, the light 10 at 390 nm was not transmitted again. In Fig. 4, when the polyvinyl chloride film 6 doped with the photochromic compound was placed in contact with the optical fibers 7 and 8, the same effect as that described above could be obtained.

A reversible filter and optical shutter can be realized by utilizing the arrangement shown in Fig. 4.

The optical circuit system shown in Fig. 5 can be also realized by employing the above arrangement. In this system, optical switches 12, 13, 14 are provided in a branched optical path 11 of optical fibers. Each of the optical switches comprises a film having the same structure as that of the above-described polyvinyl chloride film 6, the film being disposed so as to cut off the optical path. The arrangement of Fig. 5 comprises a secondary non-linear optical material 15 having the function to change incident light 16 having a wavelength of 780 nm to light 17 having a wavelength of 390 nm.

In this system, light at 260 nm is applied to each of the optical switches. When ultraviolet light at 360 nm is applied to the optical switch 12 from an irradiation apparatus 6, the optical switch 12 is turned on and transmits the light 17 at 390 nm. Namely, the optical switch 12 serves as an optical communication cable (circuit). The transmission of light to a circuit through the optical switches 13, 14 is inhibited. When the optical switch 12 is irradiated with ultraviolet light at 260 nm, the light transmission is again inhibited.

Fig. 5 shows only one concept of an optical circuit system, and the number of input optical circuits is not limited to 1, and the number of output optical circuits is not limited to 3. Optical circuits can be also formed by using known methods employing fibers, channels on a substrate, holes in a substrate or the like.

Further, in Fig. 5, the non-linear optical material can be disposed in contact with the photochromic compound.

### EXAMPLE 32

The repeatability (variability) of absorbance at 380 nm was examined by applying ultraviolet light to the dichloromethane solution, which was prepared in EXAMPLE 26 and which contained a photochromic compound.

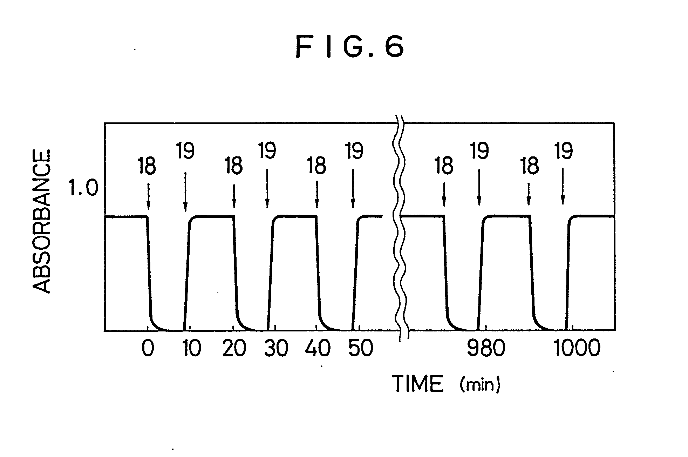

Fig. 6 shows the change with time produced when the solution was alternately irradiated with near ultraviolet light 18 at 360 nm and ultraviolet light 19 at 260 nm.

As a result, it was found that the form of the absorbance characteristic of the photochromic compound of the present invention is slightly changed after 50 times of irradiation, and that the compound is extremely excellent in its repeatability, as compared with a conventional photochromic compound, the form of the absorbance characteristic of which was changed after 5 times of irradiation.

### EXAMPLE 33

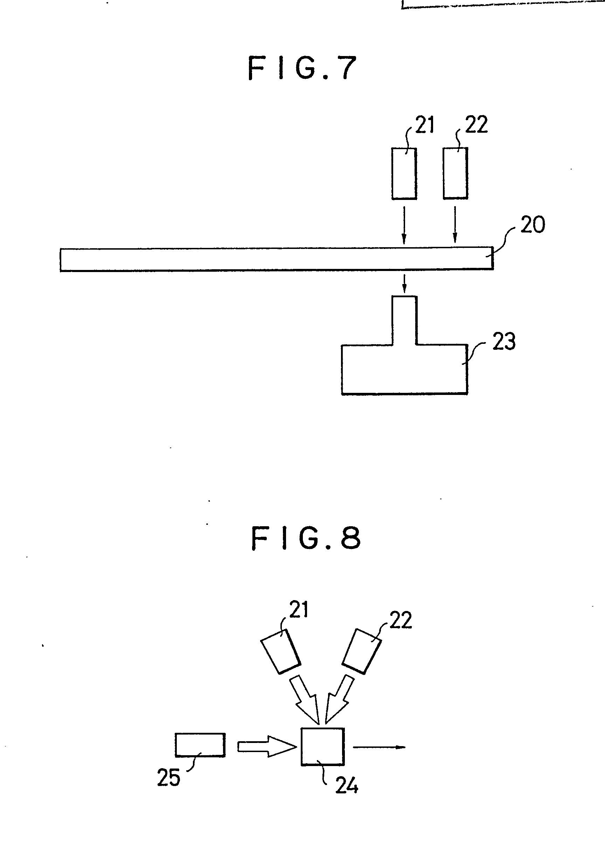

A polyvinyl chloride film 20, which was doped with 1,8-lbis(9-anthracenecarboxy)-octane and which was formed in EXAMPLE 30, was incorporated in the system shown in Fig. 7. This system comprises an apparatus 21 for applying laser light at 360 nm, an apparatus 22 for applying laser light at 260 nm, and an apparatus 23 for measuring the transmittance of the film. When laser light at 360 nm is applied to the film 20, the transmittance of a small portion (spot) of the film 20 which was irradiated is reduced. Such a reduction in absorption is recovered by irradiation with laser light at 260 nm. Since the spot can be eliminated and recovered by using laser light, the 1,8-|bis(9-anthracenecarboxy)-octane-doped polyvinyl chloride film, which was formed in EXAMPLE 30, can be used as an optical recording medium.

### EXAMPLE 34

A small piece 24 having a size of 0.3 x 0.3 mm was cut out from the 1,8-|bis(9-anthracenecarboxy)-octane-doped polyvinyl chloride film formed in EXAMPLE 30 and incorporated into the system shown in Fig. 8. This system comprises an apparatus 25 for applying laser light at 390 nm. The small piece 24 generally slightly transmits light at 390 nm. However, when laser light at 360 nm is applied to the piece 24, it transmits light at 390 nm. When laser light at 260 nm is applied to the piece 24, the piece 24 hardly transmits again light at 390 nm. The use of such an on-off property with respect to light transmission enables the system shown in Fig. 8 to be utilized as an optical switch or an optical logical element.

The present invention provides novel photosensitive compounds which undergo trans-dimerization reaction which can be controlled in a reversible manner by using only ultraviolet light.

The photosensitive compounds of the present invention are hardly affected by the influence of heat or visible light. For example, an optical recording medium composed of one of the compounds of the present invention exhibits excellent thermal stability with time and excellent resistance to repeated irradiation with ultraviolet light.

## Claims

1. Photosensitive compounds of the general formula I 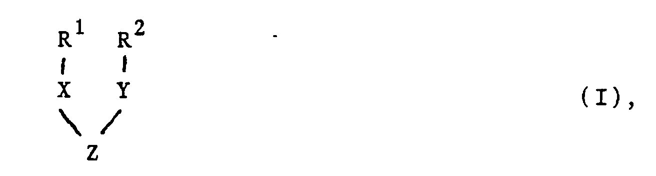 wherein denote:
- R' and R² independently an optionally substituted residue of naphthalene, anthracene, phenanthrene, triphenylene, chrysene, naphthacene, benzoanthracene, picene, pentaphene, pentacene or dibenzophenan- threne, where R' and R² may be the same if neither R' nor R² is a naphthalene residue;
- X and Y independently an ester bond, a thioester bond, an amide bond, a thioamide bond, an ether bond, a thioether bond, a sulfonyloxy bond, a sulfonylamino bond, an amine bond, or a methylene or ethylene bond, and
- Z (̵CH₂)̵ₘ(wherein m denotes an integer of 6 to 16), -CH₂(̵CH₂OCH₂)̵ₘ. CH₂- (wherein m' denotes an integer of 3 to 10), -CH₂(̵CH₂)̵ₙ-O(̵CH₂)̵ₙ: CH₂-(wherein n and n each denote an integer of 2 to 20), or a phenylene group, a xylylene group, or a cycloalkane residue having 6 or more carbon atoms, Z optionally being substituted.

2. Photosensitive compounds according to claim 1 of the following formulae (II-a), (II-b), (II-c) and (II-d): 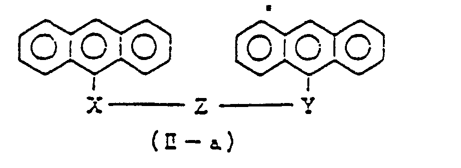 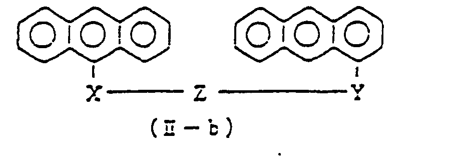 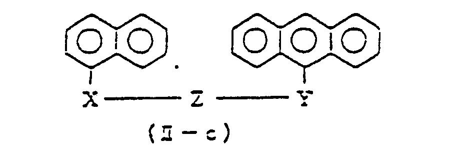 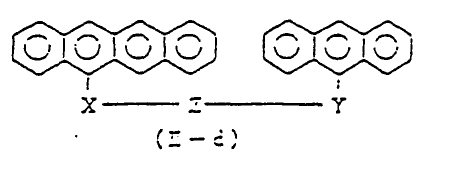 wherein X, Y and Z are defined as in claim 1.

3. Photosensitive compounds according to claim 1 or 2 of the following formulae (III-a), (III-b), (III-c), (III-d) and (III-e): 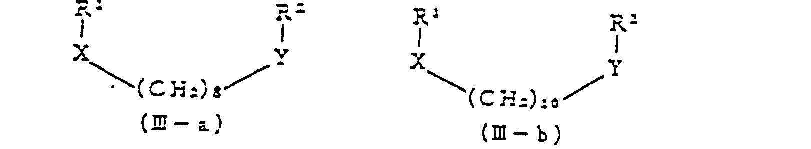 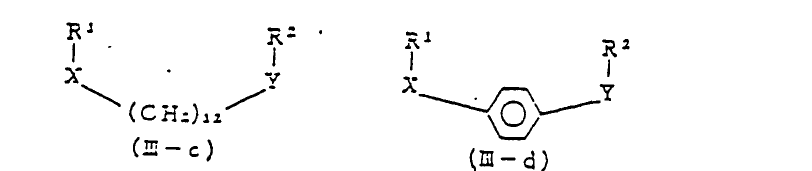 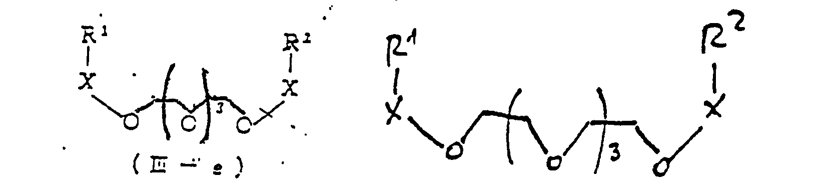 wherein R¹, R², X and Y are defined as in claim 1.

4. Photosensitive compounds according to claim 1 or 2 of the following formulae (IV-a), (IV-b), (IV-c), (IV-d) and (IV-e): 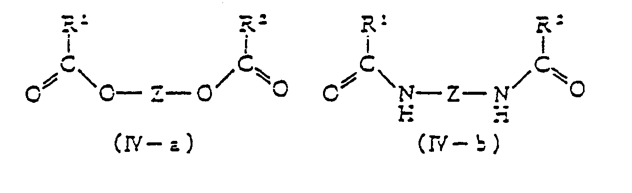 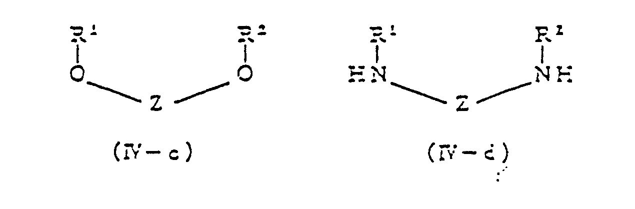 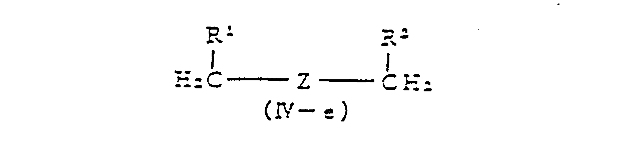 wherein R¹, R² and Z are defined as in claim 1.

5. Methods for preparing the photosensitive compounds according to one of claims 1 to 4, characterized by
(A) reacting a compound of the general formula IVa or IVb R¹-R³ (IVa) or R2-R3 (IVb), wherein
R¹ and R² are defined as in claim 1, and
R³ is -CO-OH, -CO-SH, -CS-OH, -S0₂-OH, -S0₂-Hal (Hal denoting halogen), -CO-Hal, or -CS-Hal,
or a reactive derivative of these groups, with a compound of the general formula V R⁴⁻Z-R⁴ (V), wherein
Z is defined as in claim 1, and
R⁴ is -OH, -NH₂, -Hal, or -SH, or a reactive derivative of these groups, or
(B) reacting a compound of the general formula Via or Vlb R¹-R⁶ (VIa) or R2-R5 (Vlb), wherein
R¹ and R² are defined as in claim 1, and
R⁵ is -CH₂-OH, -CH₂-Hal, -CH₂-SH, or -CH₂-NH₂,
or a reactive derivative of these groups, with a compound of the general formula VII R⁶-Z-R⁶ (VII),
wherein Z is defined as in claim 1, and
R⁶ is -COOH, -CO-SH, -CS-OH, -S0₂-OH, -S0₂-Hal, -CO-Hal, -CS-Hal, or -NH₂,
or a reactive derivative of these groups, or
(C) reacting a compound of the general formula Vllla or Vlllb 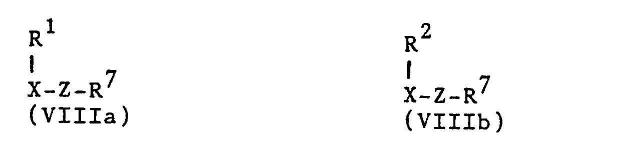 wherein
R¹, R² and Z are defined as in claim 1,
and
R⁷ is -OH, -Hal, or -NH₂, -SH,
or a reactive derivative thereof,
with a compound of the general formula IVa or IVb R¹-R³ (IVa) or R²⁻R³ (IVb) as defined above, or
(D) reacting a compound of the general formula IXa or IXb, 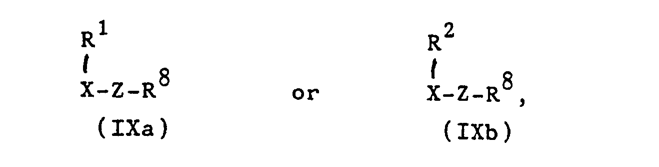 wherein
R¹, R² and Z are defined as in claim 1, and
R⁸ is -CO-OH, -CO-SH, -CS-OH, -S0₂-OH, -S0₂-Hal, -CO-Hal, or -CS-Hal,
or a reactive derivative of these groups, with a compound of the general formula Xa or Xb, R^{I-}R⁴ (Xa) or R^{z-}R⁴ (Xb), wherein
R', R² and R⁴ are as defined above.

6. Photosensitive elements having characteristics which ae reversibly changed by irradiation with light having different wavelengths, comprising a polymeric compound, which transmits light having a wavelength of 300 nm or more, and a photosensitive compound of formula I according to one of claims 1 to 4 comprised in said polymeric compound.

7. A method of producing the photosensitive elements according to claim 6, comprising the steps of dissolving a polymeric compound, which transmits light having a wavelength of 300 nm or more, and a photosensitive compound of formula I according to one of claims 1 to 4 in a solvent, and applying the obtained solution to a substrate and removing the solvent so as to incorporate the photosensitive compound in a layer of the polymeric compound.

8. Photosensitive elements having characteristics which are reversibly changed by irradiation with light having different wavelengths, comprising a substrate, which allows light having a wavelength of 300 nm or more to pass therethrough, and a photosensitive compound of formula I according to one of claims 1 to 4 which is coated on the front side and/or the rear side of the substrate.

9. A method of producing the photosensitive elements according to claim 8, comprising the steps of applying to a substrate, which allows light having a wavelength of 300 nm or more to pass therethrough, a solution of a photosensitive compound of formula I according to one of claims 1 to 4, and removing the solvent so as to form a layer of the photosensitive compound on the substrate.

10. Optical circuits comprising an optical path and an optical switch section for on-off switching the passage of light through the optical path, wherein the optical path allows light having a wavelength of 300 nm to pass therethrough and said optical switch section is provided in said optical path and composed of a photosensitive compound of formula I according to one of claims 1 to 4.

11. Optical switches and optical logical elements, for on-off switching the passage of light, comprising a supporting member which allows light having a wavelength of 300 nm or more to pass therethrough, and a photosensitive compound of formula I according to one of claims 1 to 4 supported by the supporting member.

12. Optical shutters and optical filters, for on-off switching the passage of light, being disposed in an optical path for light having a wavelength of 300 nm or more, and comprising a base material which allows light having a wavelength of 300 nm or more to pass therethrough, and a photosensitive compound of formula I according to one of claims 1 to 4, supported by the base material.

13. Optical recording medium capable of repeatedly recording and erasing by using light having different wavelengths, comprising a base material, which allows light having a wavelength of 300 nm or more to pass therethrough, and a photosensitive compound of formula I according to one of claims 1 to 4, supported by the base material.

14. Optical memory element medium capable of repeatedly recording and erasing by using light having different wavelengths, comprising a base material, which allows light having a wavelength of 300 nm or more to pass therethrough, and a photosensitive compound of formula I according to one of claims 1 to 4, supported by the base material.

15. Use of the photosensitive compounds according to claims 1 to 4 for or in photosensitive elements, optical circuits, optical switches, optical logical elements, optical filters, optical shutters, and optical recording media.

16. A method of reversibly trans-dimerizing and redissociating the photosensitive compounds of formula I according to claims 1 to 4, characterized by
- irradiation with ultraviolet light at 340 to 380 nm for trans-dimerization, and
- irradiation with ultraviolet light at 240 to 280 nm for redissociation.

17. Photosensitive compounds of formula I according to one of claims 1 to 4, characterized in that they undergo dimerization between the two aromatic ring residues in the molecule when irradiated with ultraviolet light at 340 to 380 nm, and dimerization is redissociated by irradiation with ultraviolet light at 240 to 280 nm.

18. Photosensitive compounds of formula I according to one of claims 1 to 4 and 17, characterized in that they undergo trans-dimerization between the two aromatic ring residues in the molecule when irradiated with ultraviolet light at 340 to 380 nm, and dimerization is redissociated by irradiation with ultraviolet light at 240 to 280 nm.
